# EUROPEAN PATENT SPECIFICATION

(11) **EP 3 314 257 B1**
(45) Date of publication and mention of the grant of the patent: **16.06.2021**
(21) Application number: 16736957.8
(22) Date of filing: 20.06.2016
(51) Int. Cl.: G01N 33/50, G01N 33/58, C07D 311/16, C07D 311/54, C07F 9/655, C09B 57/02

(54) **CELL TRACKING REAGENTS AND THEIR METHODS OF USE**
ZELLVERFOLGUNGSREAGENZIEN UND DEREN VERFAHREN ZUR VERWENDUNG
RÉACTIFS DE SUIVI CELLULAIRE ET LEURS PROCÉDÉS D'UTILISATION

(30) Priority: 26.06.2015 US 201562185163 P
(43) Date of publication of application: 02.05.2018
(73) Proprietor: Life Technologies Corporation, Carlsbad, CA 92008 (US)
(72) Inventor: MELQUIST, Penny, Eugene,OR 97402 (US); GEE, Kyle, Springfield,OR 97478 (US)
(74) Representative: Hoffmann Eitle
(86) International application number: PCT/US2016/038305
(87) International publication number: WO 2016/209753

(56) References cited:
- WO-A2-2007/061768
- GB-A- 2 319 250
- US-A- 6 046 016
- US-A1- 2003 228 635
- C. UTTAMAPINANT ET AL: "A fluorophore ligase for site-specific protein labeling inside living cells", PROCEEDINGS OF THE NATIONAL ACADEMY OF SCIENCES, vol. 107, no. 24, 15 June 2010 (2010-06-15), pages 10914-10919, XP055295999, US ISSN: 0027-8424, DOI: 10.1073/pnas.0914067107
- TENG CHIEH-LIN JERRY ET AL: "Mechanistic aspects of lauryl gallate-induced differentiation and apoptosis in human acute myeloid leukemia cells", FOOD AND CHEMICAL TOXICOLOGY, PERGAMON, GB, vol. 71, 30 June 2014 (2014-06-30), pages 197-206, XP029007290, ISSN: 0278-6915, DOI: 10.1016/J.FCT.2014.06.019
- YAU-TSZ LAI ET AL: "Rapid labeling of intracellular His-tagged proteins in living cells", PROCEEDINGS OF THE NATIONAL ACADEMY OF SCIENCES, vol. 112, no. 10, 23 February 2015 (2015-02-23), pages 2948-2953, XP055179061, ISSN: 0027-8424, DOI: 10.1073/pnas.1419598112
- L. COLE ET AL: "Uptake and compartmentalisation of fluorescent probes by Pisolithus tinctorius hyphae: evidence for an anion transport mechanism at the tonoplast but not for fluid-phase endocytosis", PROTOPLASMA., vol. 199, no. 1-2, 1 March 1997 (1997-03-01), pages 18-29, XP055295940, AT ISSN: 0033-183X, DOI: 10.1007/BF02539802

## Description

### FIELD

The present disclosure relates to fluorophores, including chemically-reactive fluorophores and conjugates of such fluorophores, as well as uses of such fluorophores as cell-tracking reagents.

### BACKGROUND

Methods for monitoring cell proliferation, differentiation, and function using flow cytometry have enabled investigation of complex biological phenomena, *e.g.*, immune responses to antigen, which responses involve complex interactions among multiple cell types (*see*, Wallace et al., Cytometry Part A 73A: 1019-1034 (2008)). So called cell-tracking dyes have proven useful for qualitative and quantitative monitoring of cell division, both *in vivo* and *in vitro* (*see*, Hawkins et al., Nat Protoc 2:2057-2067 (2007); and Wallace et al., Immunol Invest 36:527-561, (2007)). These dyes, also referred to herein as cell-tracking reagents or cell-tracking compounds which term is inclusive of cell-tracing reagents and cell-tracing compounds, generate a fluorescent signal that, while relatively stable in non-dividing cells, progressively decreases with each round of cell division. Reduction in fluorescence intensity can be quantified by flow cytometry in conjunction with any of several different algorithms to estimate the extent of proliferation (in response to a particular stimulus) based on dye dilution (*see*, Wallace et al., Cytometry Part A 73A: 1019-1034 (2008)). A major advantage of using flow cytometry in conjunction with cell-tracking reagents to monitor the extent of cell division is that cells can also be stained for expression of other cell surface or intracellular markers to define lineage, functionality, activation state, cytokine expression, etc. (*see,* Bercovici et al., J Immunol Methods 276:5-17, (2003); Fazekas de St Groth et al., Immunol Cell Biol 77:530-538, (1999); and Tanaka et al., Immunol Invest 33:309-324, (2004)).

Chieh-Lin Jerry Teng et al. ("Mechanistic aspects of lauryl gallate-induced differentiation and apoptosis in human acute myeloid leukemia cells", Food and Chemical Toxicology 71 (2014) 197-206) discloses the fluorescent reporter molecule 7-amino-4-trifluoromethyl coumarin for use in studying lauryl gallate induced apoptosis in human myeloid leukemia cells.

Carboxyfluorescein diacetate succinimidyl ester (CFDA-SE or, alternatively, CFSE) remains a popular, commercially available cell-tracking reagent, excitable with 488-nm laser light to give a bright green fluorescence. CFDA-SE has been widely used to monitor cell proliferation by flow cytometry in heterogeneous cell populations and stains cells with a bright homogeneous fluorescence, which is partitioned between daughter cells during each cell division.

Notwithstanding the current popularity of CFDA-SE, there remains a need for alternative fluorescent dyes, useful as cell-tracking reagents, with different spectral properties. Such reagents may be combined for simultaneous use not only with CFDA-SE, but also with other currently-available cell-analysis reagents, such as, for example, the 488 nm-excitable reagent Green Fluorescent Protein (GFP), the 635 nm-excitable red-emitting dyes allophycocyanine (APC) or ALEXA FLUOR® 633 fluorescent dye (Thermo Fisher Scientific, Waltham, MA), thereby permitting researchers to study cell proliferation, differentiation, and/or function in otherwise indistinguishable cell populations in mixed cell cultures with multi-color applications using flow cytometry.

The development of alternative cell-tracking reagents that fluoresce in the blue portion of the UV/VIS spectrum to provide bright fluorescence intensity for long-term monitoring of cell proliferation, differentiation, migration, location, and/or function using flow cytometry and/or fluorescence microscopy has, heretofore, not been realized.

### SUMMARY

Described herein are compounds, methods, and kits for short- and long-term tracking of cell proliferation. The scope of the invention is defined in the appended set of claims. The compounds disclosed herein are coumarin-based cell-tracking reagents that fluoresce in the blue portion of the UV/VIS spectrum, provide bright fluorescence intensity, uniform cell staining, good retention within cells and low toxicity toward cells. These cell-tracking reagents may be used in place of and/or in combination with other currently-available cell-analysis reagents, such as, for example, the 635 nm-excitable reagents allophycocyanine (APC) and ALEXA FLUOR® 633 fluorescent dye (Thermo Fisher Scientific, Waltham, MA) or the 488 nm-excitable Green Fluorescent Protein (GFP) to track and/or stain otherwise indistinguishable cell populations in mixed cell cultures via flow cytometry and/or fluorescence microscopy, respectively. In addition, described herein are processes for preparing the cell-tracking reagents for use in the disclosed methods and kits provided herein.

Certain embodiments provide methods for tracking cell proliferation, the methods being compatible for use with flow cytometry, wherein the methods comprise:
a) incubating a mixture of cells with a cell-tracking compound of structural formula (I): 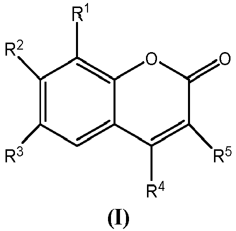 or a pharmaceutically acceptable salt thereof, wherein
   R¹ and R³ are each independently hydrogen, halogen, alkyl, or substituted alkyl;
   R² is acyl, alkoxy, substituted alkoxy, amino, substituted amino, acylamino, or hydroxyl;
   R⁴ is hydrogen, alkyl, or substituted alkyl;
   R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
   L is a linker;
   Rₓ is a reactive group; and
   S_{c} is a conjugated substance;
b) providing a stimulus to the mixture to elicit a fluorescent signal; and
c) analyzing the stimulated mixture using flow cytometry.

In certain embodiments, R¹ and R³ are each independently hydrogen or halogen. In certain embodiments, R⁴ is alkyl. In certain embodiments, R² is acyl, alkoxy, amino, substituted amino, or hydroxyl. In certain embodiments, R⁵ is -L-Rₓ. In certain embodiments, Rₓ is succinimidyl ester (SE), sulfodichlorophenyl (SDP) ester, sulfotetrafluorophenyl (STP) ester, tetrafluorophenyl (TFP) ester, and pentafluorophenyl (PFP) ester. In certain embodiments, R¹ and R³ are each independently hydrogen or halogen; R⁴ is H or alkyl; R² is acyl, alkoxy, amino, substituted amino, or hydroxyl; and R⁵ is -L-Rₓ; wherein Rₓ is succinimidyl ester (SE), sulfodichlorophenyl (SDP) ester, sulfotetrafluorophenyl (STP) ester, tetrafluorophenyl (TFP) ester, or pentafluorophenyl (PFP) ester.

In certain embodiments, Rₓ is selected from an acrylamide, an activated ester of a carboxylic acid, a carboxylic ester, an acyl azide, an acyl nitrile, an aldehyde, an alkyl halide, an anhydride, an aniline, an amine, an aryl halide, an azidc, an aziridinc, a boronatc, a diazoalkane, a haloacetamide, a haloalkyl, a halotriazine, a hydrazine, an imido ester, an isocyanate, an isothiocyanate, a maleimide, a phosphoramidite, a photoactivatable group, a reactive platinum complex, a silyl halide, a sulfonyl halide, and a thiol. In certain embodiments the reactive group is selected from the group consisting of carboxylic acid, succinimidyl ester of a carboxylic acid, hydrazide, amine and a maleimide. In certain embodiments, Rₓ is a succinimidyl ester.

In certain embodiments, S_{c} is selected from a carrier molecule and a solid support. In certain embodiments, S_{c} is selected from an amino acid, a polymer of amino acids, a peptide, a protein, a neurotoxin, a phallotoxin, a cytokine, a toxin, a protease substrate, a protein kinase substrate, an enzyme, an antibody, an antibody fragment, a lectin, a glycoprotein, a histone, an albumin, a lipoprotein, avidin, streptavidin, protein A, protein G, a phycobiliprotein, a fluorescent protein, a hormone, a growth factor, a nucleic acid base, a nucleoside, a nucleotide, a nucleic acid polymer, a nucleotide analog, a nucleoside analog, a nucleoside triphosphate, a deoxynucleoside triphosphate, a dideoxynucleoside triphosphate, a hapten, a carbohydrate, a polysaccharide, a lipid, an ion-complexing moiety (such as a crown ether), and an organic or inorganic polymer.

Certain embodiments provide methods for tracking cell proliferation, the methods being compatible for use with flow cytometry, wherein the methods comprise:
a) incubating a mixture of cells with a cell-tracking compound selected from the group consisting of: 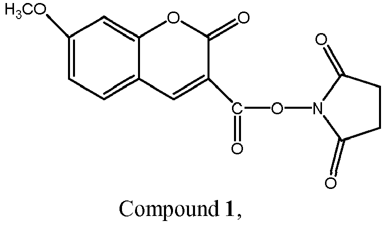 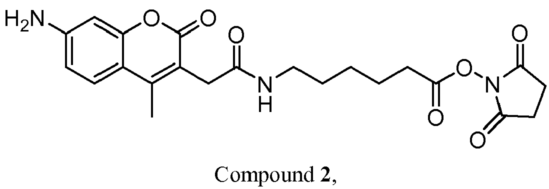 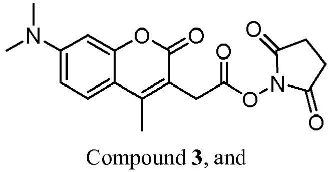 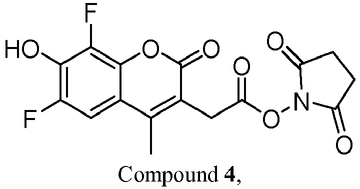 or a pharmaceutically acceptable salt thereof;
b) providing a stimulus to the mixture to elicit a fluorescent signal; and
c) analyzing the stimulated mixture using flow cytometry.

Certain embodiments provide methods for tracking cell proliferation, the methods being compatible for use with flow cytometry, wherein the methods comprise:
a) incubating a mixture of cells with a compound of the invention, or a pharmaceutically acceptable salt thereof;
b) providing a stimulus to the mixture to elicit a fluorescent signal; and
c) analyzing the stimulated mixture using flow cytometry.

In one illustrative embodiment, the methods include a second compound excitable at a different wavelength as the first compound. In another illustrative embodiment, the method includes a second compound where the second compound is APC, ALEXA FLUOR® 633 fluorescent dye (Thermo Fisher Scientific, Waltham, MA) or GFP.

In another illustrative embodiment of the methods, step a) is conducted for approximately 20 minutes. In another illustrative embodiment, step b) and step c) are carried out concurrently. In another illustrative embodiment, step b) and step c) involve flow cytometry.

In certain embodiments, compositions are provided for tracking cell proliferation, wherein the compositions comprise:
a) one or more of the cell tracker compounds of structural formula (I), or a pharmaceutically acceptable salt thereof; and
b) a carrier or an analyte,
wherein the one or more of the cell-tracker compounds are present in an amount effective to track cell proliferation.

In certain embodiments, compositions are provided for tracking cell proliferation, wherein the compositions comprise:
a) one or more of the cell tracker compounds selected from the group consisting of Compound 1, Compound 2, Compound 3, and Compound 4, or a pharmaceutically acceptable salt thereof; and
b) a carrier or an analyte,
wherein the one or more of the cell-tracker compounds are present in an amount effective to track cell proliferation

In certain embodiments, compositions are provided for tracking cell proliferation, wherein the compositions comprise:
a) one or more of the cell tracker compounds of structural formula (I), or a pharmaceutically acceptable salt thereof; and
b) a carrier or an analyte.

In certain embodiments, compositions are provided for tracking cell proliferation, wherein the compositions comprise:
a) one or more of the cell tracker compounds selected from the group consisting of Compound 1, Compound 2, Compound 3, and Compound 4, or a pharmaceutically acceptable salt thereof; and
b) a carrier or an analyte.

Certain embodiments provide a composition comprising:
a) a compound of the invention; and
b) a carrier or an analyte.

In certain embodiments, kits are provided for tracking cell proliferation, wherein the kits comprise:
(a) one or more of the cell-tracking compounds, or a pharmaceutically acceptable salt thereof described herein;
(b) one or more containers; and optionally
(c) instructions for tracking cell proliferation according to a method disclosed herein.

In certain embodiments, kits for tracking cell proliferation are provided, wherein the kit comprises:
(a) one or more of the compositions described herein;
b) an organic solvent; and
c) a desiccant.

In certain embodiments, kits are provided for tracking cell proliferation, wherein the kits comprise:
(a) one or more compounds of the invention, or a pharmaceutically acceptable salt thereof;
(b) one or more containers; and optionally
(c) instructions for tracking cell proliferation according to a method disclosed herein.

Certain embodiments provide the use of a compound, composition or kit of the invention for tracking cell proliferation.

Other illustrative aspects, features and advantages of the present disclosure will become apparent from the following detailed description. It should be understood, however, that the detailed description and the specific examples that follow, while indicating preferred embodiments arc given by way of illustration only.

### BRIEF DESCRIPTION OF THE FIGURES

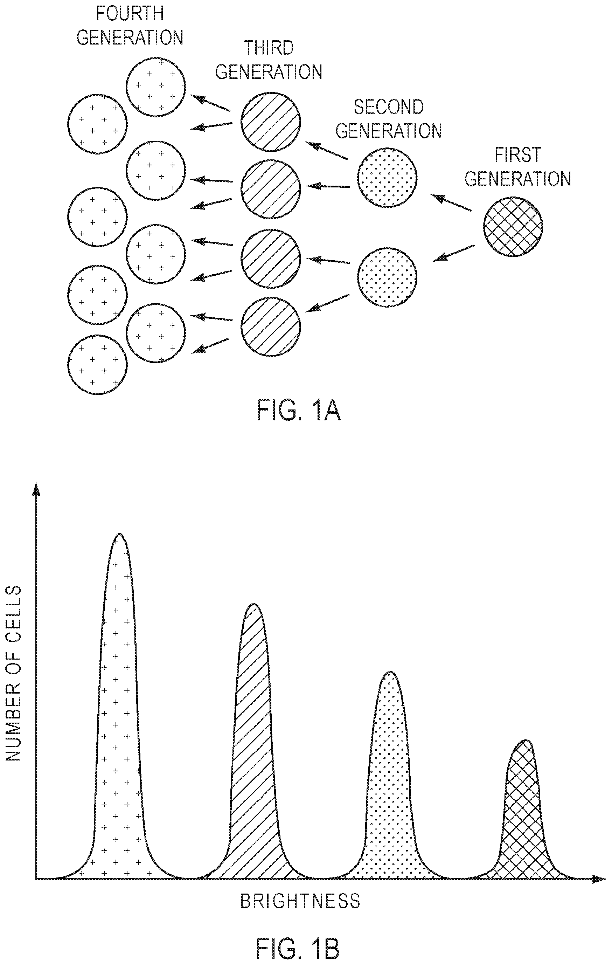

FIG. 1A and FIG. 1B: Schematic representation of the principle of cell proliferation analysis by dye dilution. A population of cells labeled with an amine-reactive cell tracking dye such as Compound 1 initially has a very bright, homogeneous fluorescent signal. FIG. 1A: As each labeled cell divides in culture, half of the fluorescent label is passed to its daughter cells, resulting in a serial halving of fluorescence intensity in each subsequent generation. FIG. 1B: Subsequent cell divisions result in a larger number of cells, each with half the fluorescence intensity of its parent cell.

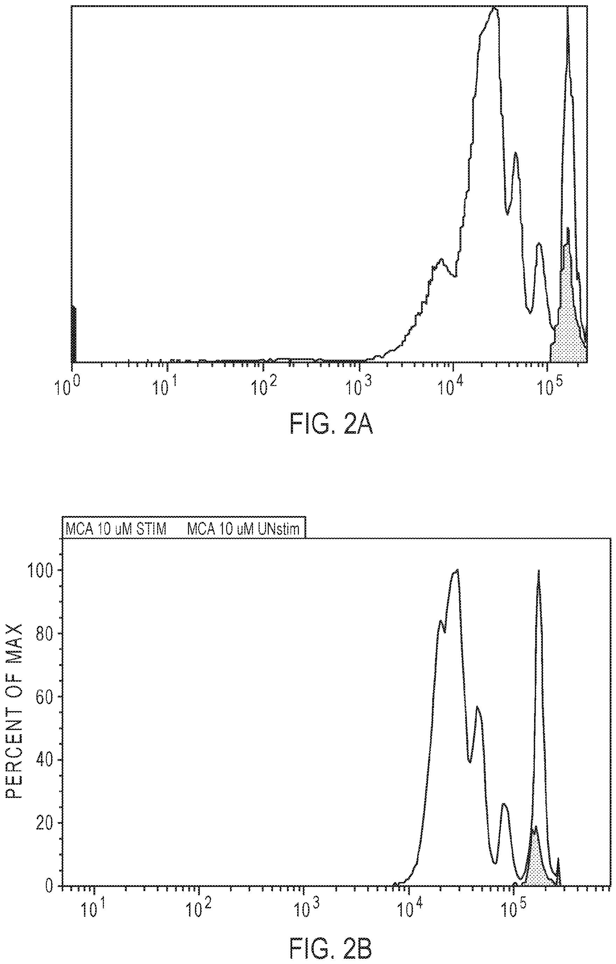

FIG. 2A and FIG. 2B: Generational tracking using Compound 1. Cell proliferation was followed for 7 days using PBMC cells incubated with Compound 1 (FIG. 2A). Cell proliferation was followed for 6 days using PBMC cells incubated with Compound 1 (FIG. 2B).

### DETAILED DESCRIPTION

The present disclosure provides compounds, methods, and kits for short- and long-term tracking of cell proliferation. The compounds of the present disclosure are cell-tracking reagents (also referred to herein as "cell-tracking compounds" which term is inclusive of cell-tracing reagents and cell-tracing compounds) that fluoresce in the blue portion of the UV/VIS spectrum and provide the following advantageous characteristics: bright fluorescence intensity, uniform cell staining, good retention within cells and have low toxicity toward cells. The cell-tracking reagents disclosed herein are coumarin-based fluorophores, including chemically-reactive fluorophores and conjugates of such fluorophores. These cell-tracking reagents may be used in place of and/or in combination with other currently-available cell-analysis reagents, such as, for example, the 488 nm-excitable Green Fluorescent Protein (GFP) and/or the 635 nm-excitable red emitting dyes allophycocyanine (APC) or ALEXA FLUOR® 633 fluorescent dye (Thermo Fisher Scientific, Waltham, MA) to track and/or stain otherwise indistinguishable cell populations in mixed cell cultures via flow cytometry and/or fluorescence microscopy, respectively. The present disclosure also includes processes for preparing and using the cell-tracking reagents described herein in the disclosed methods and kits provided herein.

### Definitions:

To more clearly and concisely describe and point out the subject matter of the present disclosure, the following definitions are provided for specific terms, which are used in the following description and appended claims. Throughout the specification, exemplification of specific terms should be considered as non-limiting examples.

Before describing the present teachings in detail, it is to be understood that the disclosure is not limited to specific compositions or process steps, as such may vary. It should be noted that, as used in this specification and the appended claims, the singular form "a", "an" and "the" include plural references unless the context clearly dictates otherwise. Thus, for example, reference to "a cell-tracking reagent" includes a plurality of cell-tracking reagents and reference to "a cell" includes a plurality of cells and the like. The phrase "and/or" denotes a shorthand way of indicating that the specific combination is contemplated in combination and separately, in the alternative. For illustration purposes, but not as a limitation, "X and/or Y" can mean "X" or "Y" or "X" and "Y".

It will be appreciated that there is an implied "about" prior to the temperatures, concentrations, times, *etc.* discussed in the present disclosure, such that slight and insubstantial deviations are within the scope of the present teachings herein. Also, the use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the teachings.

Unless specifically noted in the above specification, embodiments in the specification that recite "comprising" various components are also contemplated as "consisting of" or "consisting essentially of" the recited components; embodiments in the specification that recite "consisting of" various components are also contemplated as "comprising" or "consisting essentially of" the recited components; and embodiments in the specification that recite "consisting essentially of" various components are also contemplated as "consisting of" or "comprising" the recited components (this interchangeability does not apply to the use of these terms in the claims).

The term "or combinations thereof" as used herein refers to all permutations and combinations of the listed terms preceding the term. For example, "A, B, C, or combinations thereof" is intended to include at least one of: A, B, C, AB, AC, BC, or ABC, and if order is important in a particular context, also BA, CA, CB, ACB, CBA, BCA, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

A dashed line projecting from a substituent, such as: 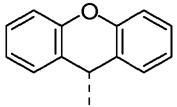 indicates the point of attachment to the base molecule. For a fused ring, dashed lines indicate portions of the base molecule where the fused ring is attached, such as: 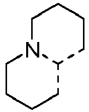 wherein the full molecule could have the structure:

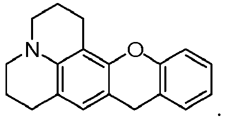

Unless indicated otherwise, the nomenclature of substituents that are not explicitly defined herein are arrived at by naming the terminal portion of the functionality followed by the adjacent functionality toward the point of attachment. For example, the substituent "arylalkyloxycarbonyl" refers to the group (aryl)-(alkyl)-O-C(O)-.

It is understood that in all substituted groups defined herein, polymers arrived at by defining substituents with further substituents to themselves (*e.g.*, substituted aryl having a substituted aryl group as a substituent which is itself substituted with a substituted aryl group, which is further substituted by a substituted aryl group *etc*.) are not intended for inclusion herein. In such cases, the maximum number of such substitutions is three. For example, serial substitutions of substituted aryl groups with two other substituted aryl groups are limited to -substituted aryl-(substituted aryl)-substituted aryl.

Similarly, it is understood that the definitions provided herein are not intended to include impermissible substitution patterns (*e.g.*, methyl substituted with 5 fluoro groups). Such impermissible substitution patterns are well known to the skilled artisan.

The cell-tracking compounds disclosed herein may exist in unsolvated forms as well as solvated forms, including hydrated forms. These compounds may exist in multiple crystalline or amorphous forms. In general, all physical forms are equivalent for the uses described herein and are intended to be within the scope of the present disclosure. The cell-tracking compounds disclosed herein may possess asymmetric carbon atoms (*i.e.*, chiral centers) or double bonds; the racemates, diastereomers, geometric isomers and individual isomers of the cell-tracking compounds described herein are within the scope of the present disclosure. The cell tracking compounds described herein may be prepared as a single isomer or as a mixture of isomers.

Where substituent groups are specified by their conventional chemical formulae and are written from left to right, they equally encompass the chemically identical substituents, which would result from writing the structure from right to left, *e.g.*, -CH₂O- is intended to also recite -OCH₂-.

It will be understood that the chemical structures that are used to define the cell-tracking compounds disclosed herein are each representations of one of the possible resonance structures by which each given structure can be represented. Further, it will be understood that by definition, resonance structures are merely a graphical representation used by those of skill in the art to represent electron delocalization, and that the present disclosure is not limited in any way by showing one particular resonance structure for any given structure.

Where a disclosed compound includes a conjugated ring system, resonance stabilization may permit a formal electronic charge to be distributed over the entire molecule. While a particular charge may be depicted as localized on a particular ring system, or a particular heteroatom, it is commonly understood that a comparable resonance structure can be drawn in which the charge may be formally localized on an alternative portion of the compound.

As used herein, the terms "cell-tracking reagent" and "cell-tracking compound," which terms are inclusive of cell-tracing reagents and cell-tracing compounds, are used interchangeably and refer to the coumarin-based compounds described herein.

"Alkyl" refers to monovalent saturated aliphatic hydrocarbyl groups having from 1 to 10 carbon atoms and preferably 1 to 6 carbon atoms, e.g. 1, 2, 3, 4, 5 or 6 carbon atoms. This term includes, by way of example, linear and branched hydrocarbyl groups such as methyl (CH₃-), ethyl (CH₃CH₂-), *n*-propyl (CH₃CH₂CH₂-), isopropyl ((CH₃)₂CH-), *n*-butyl (CH₃CH₂CH₂CH₂-), isobutyl ((CH₃)₂CHCH₂-), sec-butyl ((CH₃)(CH₃CH₂)CH-), *t*-butyl ((CH₃)₃C-), n-pentyl (CH₃CH₂CH₂CH₂CH₂-), and neopentyl ((CH₃)₃CCH₂-).

"Substituted alkyl" refers to an alkyl group having from 1 to 5, preferably 1 to 3, or more preferably 1 to 2 substituents selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxylalkyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, sulfonate (*e.g.*, -SO₃H, -SO₃⁻), substituted sulfonyl, sulfonyloxy, thioacyl, thiol, alkylthio, and substituted alkylthio, wherein said substituents are defined herein. Particular substituted alkyl groups comprise a reactive group for direct or indirect linking to a carrier molecule or solid support, for example, but not limited to, alkyl substituted by carboxyl or a carboxyl ester (*e.g.* an activated ester such as an N-hydroxysuccinimide ester) and alkyl substituted by aminocarbonyl -CONHR where R is an organic moiety as defined below with reference to the term "aminocarbonyl", *e.g.* a C₁-C₁₀ (*e.g.* C₁-C₆) alkyl terminally substituted by a reactive group (Rₓ) including, but not limited to, carboxyl, carboxylester, maleimide, succinimidyl ester (SE), sulfodichlorophenyl (SDP) ester, sulfotetrafluorophenyl (STP) ester, tetrafluorophenyl (TFP) ester, pentafluorophenyl (PFP) ester, nitrilotriacetic acid (NTA), aminodextran, and cyclooctyne-amine.

"Alkoxy" refers to the group -O-alkyl wherein alkyl is defined herein. Alkoxy includes, by way of example, methoxy, ethoxy, n-propoxy, isopropoxy, *n*-butoxy, *t*-butoxy, sec-butoxy, and n-pentoxy.

"Substituted alkoxy" refers to the group -O-(substituted alkyl), wherein substituted alkyl is defined herein.

"Aryl" or "Ar" refers to a monovalent aromatic carboxylic group of from 6 to 14 carbon atoms having a single ring (*e.g*., phenyl) or multiple condensed rings (*e.g.*, naphthyl or anthryl) which condensed rings may or may not be aromatic (*e.g.*, 2-benzoxazolinone, 2H-1,4-benzoxazin-3(4H)-one-7-yl, and the like) provided that the point of attachment is at an aromatic carbon atom. Preferred aryl groups include phenyl and naphthyl.

"Substituted aryl" refers to aryl groups which are substituted with 1 to 5, preferably 1 to 3, or more preferably 1 to 2 substituents selected from the group consisting of alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, sulfonate (*e.g.*, -SO₃H, -SO₃⁻), substituted sulfonyl, sulfonyloxy, thioacyl, thiol, alkylthio, and substituted alkylthio, wherein said substituents are defined herein.

"Heteroaryl" refers to an aromatic group of from 1 to 10 carbon atoms and 1 to 4 heteroatoms selected from the group consisting of oxygen, nitrogen and sulfur within the ring. Such heteroaryl groups can have a single ring (*e.g.*, pyridinyl or furyl) or multiple condensed rings (*e.g.*, indolizinyl or benzothienyl) wherein the condensed rings may or may not be aromatic and/or contain a heteroatom provided that the point of attachment is through an atom of the aromatic heteroaryl group. In one embodiment, the nitrogen and/or the sulfur ring atom(s) of the heteroaryl group are optionally oxidized to provide for the N-oxide (N→O), sulfinyl, or sulfonyl moieties. Preferred heteroaryls include pyridinyl, pyrrolyl, indolyl, thiophenyl, and furanyl.

"Substituted heteroaryl" refers to heteroaryl groups that are substituted with from 1 to 5, preferably 1 to 3, or more preferably 1 to 2 substituents selected from the group consisting of the same group of substituents defined for substituted aryl.

"Heteroaryloxy" refers to -O-heteroaryl.

"Substituted heteroaryloxy" refers to the group -O-(substituted heteroaryl).

"Alkenyl" refers to alkenyl groups having from 2 to 6 carbon atoms and preferably 2 to 4 carbon atoms and having at least 1 and preferably from 1 to 2 sites of alkenyl unsaturation. Such groups are exemplified, for example, by vinyl, allyl, but-3-en-1-yl, and propenyl.

"Substituted alkenyl" refers to alkenyl groups having from 1 to 3 substituents, and preferably 1 to 2 substituents, selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, sulfonate (*e.g.*, -SO₃H, -SO₃⁻), substituted sulfonyl, sulfonyloxy, thioacyl, thiol, alkylthio, and substituted alkylthio, wherein said substituents are defined herein and with the proviso that any hydroxy substitution is not attached to a vinyl (unsaturated) carbon atom.

"Acyl" refers to the groups H-C(O)-, alkyl-C(O)-, substituted alkyl-C(O)-, alkenyl-C(O)-, substituted alkenyl-C(O)-, alkynyl-C(O)-, substituted alkynyl-C(O)-, cycloalkyl-C(O)-, substituted cycloalkyl-C(O)-, cycloalkenyl-C(O)-, substituted cycloalkenyl-C(O)-, aryl-C(O)-, substituted aryl-C(O)-, heteroaryl-C(O)-, substituted heteroaryl-C(O)-, heterocyclic-C(O)-, and substituted heterocyclic-C(O)-, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein. Acyl includes the "acetyl" group CH₃C(O)-.

"Acylamino" refers to the groups -NRC(O)alkyl, -NRC(O)substituted alkyl, -NRC(O)cycloalkyl, -NRC(O)substituted cycloalkyl, -NRC(O)cycloalkenyl, -NRC(O)substituted cycloalkenyl, -NRC(O)alkenyl, -NRC(O)substituted alkenyl, -NRC(O)alkynyl, -NRC(O)substituted alkynyl, -NRC(O)aryl, -NRC(O)substituted aryl, -NRC(O)heteroaryl, -NRC(O)substituted heteroaryl, -NRC(O)heterocyclic, and -NRC(O)substituted heterocyclic, wherein R is hydrogen or alkyl and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Acyloxy" refers to the groups alkyl-C(O)O-, substituted alkyl-C(O)O-, alkenyl-C(O)O-, substituted alkenyl-C(O)O-, alkynyl-C(O)O-, substituted alkynyl-C(O)O-, aryl-C(O)O-, substituted aryl-C(O)O-, cycloalkyl-C(O)O-, substituted cycloalkyl-C(O)O-, cycloalkenyl-C(O)O-, substituted cycloalkenyl-C(O)O-, heteroaryl-C(O)O-, substituted heteroaryl-C(O)O-, heterocyclic-C(O)O-, and substituted heterocyclic-C(O)O-, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Amino" refers to the group -NH₂.

"Substituted amino" refers to the group -NR'R" where R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, substituted heterocyclic, -SO₂-alkyl, -SO₂-substituted alkyl, -SO₂-alkenyl, -SO₂-substituted alkenyl, -SO₂-cycloalkyl, -SO₂-substituted cycloalkyl, -SO₂-cycloalkenyl, -SO₂-substituted cylcoalkenyl,-SO₂-aryl, -SO₂-substituted aryl, -SO₂-heteroaryl, -SO₂-substituted heteroaryl, -SO₂-heterocyclic, and -SO₂-substituted heterocyclic and wherein R' and R" are optionally joined, together with the nitrogen bound thereto to form a heterocyclic or substituted heterocyclic group, provided that R' and R" are both not hydrogen, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein. When R' is hydrogen and R" is alkyl, the substituted amino group is sometimes referred to herein as alkylamino. When R' and R" are alkyl, the substituted amino group is sometimes referred to herein as dialkylamino. When referring to a monosubstituted amino, it is meant that either R' or R" is hydrogen but not both. When referring to a disubstituted amino, it is meant that neither R' nor R" are hydrogen.

"Aminocarbonyl" refers to the group -C(O)NR'R" where R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Aminothiocarbonyl" refers to the group -C(S)NR'R" where R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Aminocarbonylamino" refers to the group -NRC(O)NR'R" where R is hydrogen or alkyl and R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Aminothiocarbonylamino" refers to the group -NRC(S)NR'R" where R is hydrogen or alkyl and R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Aminocarbonyloxy" refers to the group -O-C(O)NR'R" where R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Aminosulfonyl" refers to the group -SO₂NR'R" where R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Aminosulfonyloxy" refers to the group -O-SO₂NR'R" where R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Aminosulfonylamino" refers to the group -NRSO₂NR'R" where R is hydrogen or alkyl and R' and R" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Amidino" refers to the group -C(=NR"')R'R" where R', R", and R'" are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where R' and R" are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Aryloxy" refers to the group -O-aryl, where aryl is as defined herein, that includes, by way of example, phenoxy and naphthoxy.

"Substituted aryloxy" refers to the group -O-(substituted aryl), where substituted aryl is as defined herein.

"Arylthio" refers to the group -S-aryl, where aryl is as defined herein.

"Substituted arylthio" refers to the group -S-(substituted aryl), where substituted aryl is as defined herein.

"Alkynyl" refers to alkynyl groups having from 2 to 6 carbon atoms and preferably 2 to 3 carbon atoms and having at least 1 and preferably from 1 to 2 sites of alkynyl unsaturation.

"Substituted alkynyl" refers to alkynyl groups having from 1 to 3 substituents, and preferably 1 to 2 substituents, selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, -SO₃⁻, substituted sulfonyl, sulfonyloxy, thioacyl, thiol, alkylthio, and substituted alkylthio, wherein said substituents are defined herein and with the proviso that any hydroxy substitution is not attached to an acetylenic carbon atom.

"Carbonyl" refers to the divalent group -C(O)- which is equivalent to -C(=O)-.

"Carboxyl" or "carboxy" refers to -COOH or salts thereof.

"Carboxyl alkyl" or "carboxyalkyl" refers to the group -(CH₂)ₙCOOH, where n is 1-6.

"Carboxyl ester" or "carboxy ester" refers to the groups -C(O)O-alkyl, -C(O)O-substituted alkyl, -C(O)O-alkenyl, -C(O)O-substituted alkenyl, -C(O)O-alkynyl, -C(O)O-substituted alkynyl, -C(O)O-aryl, -C(O)O-substituted aryl, -C(O)O-cycloalkyl, -C(O)O-substituted cycloalkyl, -C(O)O-cycloalkenyl, -C(O)O-substituted cycloalkenyl, -C(O)O-heteroaryl, -C(O)O-substituted heteroaryl, -C(O)O-heterocyclic, and -C(O)O-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"(Carboxyl ester)amino" refers to the group -NR-C(O)O-alkyl, -NR-C(O)O-substituted alkyl, -NR-C(O)O-alkenyl, -NR-C(O)O-substituted alkenyl, -NR-C(O)O-alkynyl, -NR-C(O)O-substituted alkynyl, -NR-C(O)O-aryl, -NR-C(O)O-substituted aryl, -NR-C(O)O-cycloalkyl, -NR-C(O)O-substituted cycloalkyl, -NR-C(O)O-cycloalkenyl, - NR-C(O)O-substituted cycloalkenyl, -NR-C(O)O-heteroaryl, -NR-C(O)O-substituted heteroaryl, -NR-C(O)O-heterocyclic, and -NR-C(O)O-substituted heterocyclic, wherein R is alkyl or hydrogen, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"(Carboxyl ester)oxy" refers to the group -O-C(O)O-alkyl, -O-C(O)O-substituted alkyl, -O-C(O)O-alkenyl, -O-C(O)O-substituted alkenyl, -O-C(O)O-alkynyl, -O-C(O)O-substituted alkynyl, -O-C(O)O-aryl, -O-C(O)O-substituted aryl, -O-C(O)O-cycloalkyl, -O-C(O)O-substituted cycloalkyl, -O-C(O)O-cycloalkenyl, -O-C(O)O-substituted cycloalkenyl, -O-C(O)O-heteroaryl, -O-C(O)O-substituted heteroaryl, -O-C(O)O-heterocyclic, and -O-C(O)O-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Cyano" refers to the group -CN.

"Cycloalkyl" refers to cyclic alkyl groups of from 3 to 10 carbon atoms having single or multiple cyclic rings including fused, bridged, and spiro ring systems. Examples of suitable cycloalkyl groups include, for instance, adamantyl, cyclopropyl, cyclobutyl, cyclopentyl, and cyclooctyl.

"Cycloalkenyl" refers to non-aromatic cyclic alkyl groups of from 3 to 10 carbon atoms having single or multiple cyclic rings and having at least one >C=C< ring unsaturation and preferably from 1 to 2 sites of >C=C< ring unsaturation.

"Substituted cycloalkyl" and "substituted cycloalkenyl" refer to a cycloalkyl or cycloalkenyl group having from 1 to 5 or preferably 1 to 3 substituents selected from the group consisting of oxo, thione, alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, sulfonate (*e.g.*, -SO₃H, -SO₃⁻), substituted sulfonyl, sulfonyloxy, thioacyl, thiol, alkylthio, and substituted alkylthio, wherein said substituents are defined herein.

"Cycloalkyloxy" refers to -O-cycloalkyl.

"Substituted cycloalkyloxy" refers to -O-(substituted cycloalkyl).

"Cycloalkylthio" refers to -S-cycloalkyl.

"Substituted cycloalkylthio" refers to -S-(substituted cycloalkyl).

"Cycloalkenyloxy" refers to -O-cycloalkenyl.

"Substituted cycloalkenyloxy" refers to -O-(substituted cycloalkenyl).

"Cycloalkenylthio" refers to -S-cycloalkenyl.

"Substituted cycloalkenylthio" refers to -S-(substituted cycloalkenyl).

"Guanidino" refers to the group -NHC(=NH)NH₂.

"Substituted guanidino" refers to -NR¹³C(=NR¹³)N(R¹³)₂ where each R¹³ is independently selected from the group consisting of hydrogen, alkyl, substituted alkyl, aryl, substituted aryl, heteroaryl, substituted heteroaryl, heterocyclic, and substituted heterocyclic and two R¹³ groups attached to a common guanidino nitrogen atom are optionally joined together with the nitrogen bound thereto to form a heterocyclic or substituted heterocyclic group, provided that at least one R¹³ is not hydrogen, and wherein said substituents are as defined herein.

"H" indicates hydrogen.

"Halo" or "halogen" refers to fluoro, chloro, bromo and iodo.

"Hydroxy" or "hydroxyl" refers to the group -OH.

"Heteroaryl" refers to an aromatic group of from 1 to 10 carbon atoms and 1 to 4 heteroatoms selected from the group consisting of oxygen, nitrogen and sulfur within the ring. Such heteroaryl groups can have a single ring (*e.g.*, pyridinyl or furyl) or multiple condensed rings (*e.g.*, indolizinyl or benzothienyl) wherein the condensed rings may or may not be aromatic and/or contain a heteroatom provided that the point of attachment is through an atom of the aromatic heteroaryl group. In one embodiment, the nitrogen and/or the sulfur ring atom(s) of the heteroaryl group are optionally oxidized to provide for the N-oxide (N→O), sulfinyl, or sulfonyl moieties. Preferred heteroaryls include pyridinyl, pyrrolyl, indolyl, thiophenyl, and furanyl.

"Substituted heteroaryl" refers to heteroaryl groups that are substituted with from 1 to 5, preferably 1 to 3, or more preferably 1 to 2 substituents selected from the group consisting of the same group of substituents defined for substituted aryl.

"Heteroaryloxy" refers to -O-heteroaryl.

"Substituted heteroaryloxy" refers to the group -O-(substituted heteroaryl).

"Heteroarylthio" refers to the group -S-heteroaryl.

"Substituted heteroarylthio" refers to the group -S-(substituted heteroaryl).

"Heterocycle" or "heterocyclic" or "heterocycloalkyl" or "heterocyclyl" refers to a saturated or unsaturated group having a single ring or multiple condensed rings, including fused bridged and spiro ring systems, from 1 to 10 carbon atoms and from 1 to 4 hetero atoms selected from the group consisting of nitrogen, sulfur or oxygen within the ring wherein, in fused ring systems, one or more the rings can be cycloalkyl, aryl or heteroaryl provided that the point of attachment is through the non-aromatic ring. In one embodiment, the nitrogen and/or sulfur atom(s) of the heterocyclic group are optionally oxidized to provide for the N-oxide, sulfinyl, and sulfonyl moieties.

"Substituted heterocyclic" or "substituted heterocycloalkyl" or "substituted heterocyclyl" refers to heterocyclyl groups that are substituted with from 1 to 5, or preferably 1 to 3 of the same substituents as defined for substituted cycloalkyl.

Examples of heterocycles and heteroaryls include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, and tetrahydrofuranyl.

"Heterocyclyloxy" refers to the group -O-heterocyclyl.

"Substituted heterocyclyloxy" refers to the group -O-(substituted heterocyclyl).

"Heterocyclylthio" refers to the group -S- heterocyclyl.

"Substituted heterocyclylthio" refers to the group -S-(substituted heterocyclyl).

Examples of heterocycle and heteroaryls include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, and tetrahydrofuranyl.

"Hydrazinyl" refers to the group -NHNH₂- or =NNH-.

"Substituted hydrazinyl" refers to a hydrazinyl group, wherein a non-hydrogen atom, such as an alkyl group, is appended to one or both of the hydrazinyl amine groups. An example of substituted hydrazinyl is -N(alkyl)-NH₂ or =N⁺(alkyl)-NH₂.

"Nitro" refers to the group -NO₂.

"Oxo" refers to the atom (=O) or (-O⁻).

"Spirocyclyl" refers to divalent saturated cyclic group from 3 to 10 carbon atoms having a cycloalkyl or heterocyclyl ring with a spiro union (the union formed by a single atom which is the only common member of the rings) as exemplified by the following structure:

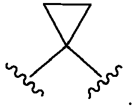

"Sulfonyl" refers to the divalent group -S(O)₂-.

"Substituted sulfonyl" refers to the group -SO₂-alkyl, -SO₂-substituted alkyl, -SO₂-alkenyl, -SO₂-substituted alkenyl, -SO₂-cycloalkyl, -SO₂-substituted cycloalkyl, -SO₂-cycloalkenyl, -SO₂-substituted cycloalkenyl, -SO₂-aryl, -SO₂-substituted aryl, -SO₂-heteroaryl, -SO₂-substituted heteroaryl, -SO₂-heterocyclic, -SO₂-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein. Substituted sulfonyl includes groups such as methyl-SO₂-, phenyl-SO₂-, and 4-methylphenyl-SO₂-.

"Sulfonyloxy" refers to the group -OSO₂-alkyl, -OSO₂-substituted alkyl, -OSO₂-alkenyl, -OSO₂-substituted alkenyl, -OSO₂-cycloalkyl, -OSO₂-substituted cycloalkyl, -OSO₂-cycloalkenyl, -OSO₂-substituted cylcoalkenyl,-OSO₂-aryl, -OSO₂-substituted aryl, -OSO₂-heteroaryl, -OSO₂-substituted heteroaryl, -OSO₂-heterocyclic, -OSO₂-substituted heterocyclic, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Thioacyl" refers to the groups H-C(S)-, alkyl-C(S)-, substituted alkyl-C(S)-, alkenyl-C(S)-, substituted alkenyl-C(S)-, alkynyl-C(S)-, substituted alkynyl-C(S)-, cycloalkyl-C(S)-, substituted cycloalkyl-C(S)-, cycloalkenyl-C(S)-, substituted cycloalkenyl-C(S)-, aryl-C(S)-, substituted aryl-C(S)-, heteroaryl-C(S)-, substituted heteroaryl-C(S)-, heterocyclic-C(S)-, and substituted heterocyclic-C(S)-, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein.

"Thiol" refers to the group -SH.

"Thiocarbonyl" refers to the divalent group -C(S)- which is equivalent to -C(=S)-.

"Thione" refers to the atom (=S).

"Alkylthio" refers to the group -S-alkyl wherein alkyl is as defined herein.

"Substituted alkylthio" refers to the group -S-(substituted alkyl), wherein substituted alkyl is as defined herein.

The term "carrier molecule" as used herein, refers to a biological or a non-biological component that is or becomes covalently bonded to a cell-tracker compound disclosed herein. Such components include, but are not limited to, an amino acid, a peptide, a protein, a polysaccharide, a nucleoside, a nucleotide, an oligonucleotide, a nucleic acid, a hapten, a psoralen, a drug, a hormone, a lipid, a lipid assembly, a synthetic polymer, a polymeric microparticle, a biological cell, a virus and combinations thereof. Included is one embodiment in which carrier molecules comprise an organic moiety having at least 4 plural valent atoms and often more than 10 plural valent atoms (*i.e*., atoms other than hydrogen and halo), *e.g.* at least 15 such atoms, as in the case of moieties having at least 20 such atoms.

The term "conjugated substance" or "S_{c}" refers to a carrier molecule or solid support.

The term "detectable response" as used herein refers to an occurrence of or a change in, a signal that is directly or indirectly detectable either by observation or by instrumentation. Typically, the detectable response is an optical response resulting in a change in the wavelength distribution patterns or intensity of absorbance or fluorescence or a change in light scatter, fluorescence lifetime, fluorescence polarization, or a combination of the above parameters.

The term "dye" as used herein refers to a compound that emits light to produce an observable detectable signal.

As used herein, the term "fluorophore" or "fluorogenic" refers to a compound or a composition that demonstrates a change in fluorescence upon binding to a biological compound or analyte of interest and/or upon cleavage by an enzyme. The fluorophores of the present disclosure may be substituted to alter the solubility, spectral properties or physical properties of the fluorophore.

As used herein, "a pharmaceutically acceptable salt" or "a biologically compatible salt" is a counterion that is not toxic as used, and does not have a substantially deleterious effect on biomolecules. Examples of such salts include, among others, K⁺, Na⁺, Cs⁺, Li⁺, Ca²⁺, Mg²⁺, Cl⁻, AcO⁻, and alkylammonium or alkoxyammonium salts.

The term "linker" or "L", as used herein, refers to a single covalent bond or a moiety comprising series of stable covalent bonds, the moiety often incorporating 1-40 plural valent atoms selected from the group consisting of C, N, O, S and P that covalently attach the fluorogenic or fluorescent compounds to another moiety such as a chemically reactive group or a biological and non-biological component. The number of plural valent atoms in a linker may be, for example, 0, 1, 2, 3, 4, 5, 6, 7, 8, 9, 10, 20, 25, 30 or a larger number up to 40 or more. A linker may be linear or non-linear; some linkers have pendant side chains or pendant functional groups, or both. Examples of such pendant moieties are hydrophilicity modifiers, for example solubilizing groups like, e.g. sulfonate (-SO₃H or -SO₃⁻). In certain embodiments, L is composed of any combination of single, double, triple or aromatic carbon-carbon bonds, carbon-nitrogen bonds, nitrogen-nitrogen bonds, carbon-oxygen bonds and carbon-sulfur bonds. Exemplary linking members include a moiety that includes -C(O)NH-, -C(O)O-, -NH-, -S-, -O-, and the like. Linkers may, by way of example, consist of a combination of moieties selected from alkyl; -C(O)NH-; -C(O)O-; -NH-; -S-; -O-; -C(O)-; -S(O)ₙ- where n is 0, 1 or 2; -O-; 5- or 6-membered monocyclic rings; and optional pendant functional groups, for example sulfo, hydroxy and carboxy. The moiety formed by a linker bonded to a reactive group (Rₓ) may be designated -L-Rₓ. The reactive group may be reacted with a substance reactive therewith, whereby the linker becomes bonded to a conjugated substance (S_{c}) and may be designated -L-S_{c}, or in some cases, the linker may contains a residue of a reactive group (*e.g.* the carbonyl group of an ester) and may be designated "-L_{R}". A "cleavable linker" is a linker that has one or more cleavable groups that may be broken by the result of a reaction or condition. The term "cleavable group" refers to a moiety that allows for release of a portion, *e.g.*, a fluorogenic or fluorescent moiety, of a conjugate from the remainder of the conjugate by cleaving a bond linking the released moiety to the remainder of the conjugate. Such cleavage is either chemical in nature, or enzymatically mediated. Exemplary enzymatically cleavable groups include natural amino acids or peptide sequences that end with a natural amino acid.

In addition to enzymatically cleavable groups, it is within the scope of the present disclosure to include one or more sites that are cleaved by the action of an agent other than an enzyme. Exemplary non-enzymatic cleavage agents include, but are not limited to, acids, bases, light (*e.g.*, nitrobenzyl derivatives, phenacyl groups, benzoin esters), and heat. Many cleavable groups are known in the art. *See*, for example, Jung et al., Biochem. Biophys. Acta, 761:152-162 (1983); Joshi et al., J. Biol. Chem., 265:14518-14525 (1990); Zarling et al., J. Immunol., 124:913-920 (1980); Bouizar et al., Eur. J. Biochem., 155:141-147 (1986); Park et al., J. Biol. Chem., 261:205-210 (1986); Browning et al., J. Immunol., 143:1859-1867 (1989). Moreover a broad range of cleavable, bifunctional (both homo- and hetero-bifunctional) spacer arms are commercially available.

An exemplary cleavable group, such as an ester, is a cleavable group that may be cleaved by a reagent, *e.g.*, sodium hydroxide, resulting in a carboxylate-containing fragment and a hydroxyl-containing product.

The linker may be used to attach the cell-tracker compound to another component of a conjugate, such as a targeting moiety (*e.g.*, antibody, ligand, non-covalent protein-binding group, *etc.*), an analyte, a biomolecule, a drug and the like.

The term "reactive group" (or "Rₓ"), as used herein, refers to a group that is capable of reacting with another chemical group to form a covalent bond, *i.e*., is covalently reactive under suitable reaction conditions, and generally represents a point of attachment for another substance. The reactive group is a moiety, such as carboxylic acid or succinimidyl ester, on the compounds of the present disclosure that is capable of chemically reacting with a functional group on a different compound to form a covalent linkage. Reactive groups generally include nucleophiles, electrophiles and photoactivatable groups.

Exemplary reactive groups include, but not limited to, olefins, acetylenes, alcohols, phenols, ethers, oxides, halides, aldehydes, ketones, carboxylic acids, esters, amides, cyanates, isocyanates, thiocyanates, isothiocyanates, amines, hydrazines, hydrazones, hydrazides, diazo, diazonium, nitro, nitriles, mercaptans, sulfides, disulfides, sulfoxides, sulfones, sulfonic acids, sulfinic acids, acetals, ketals, anhydrides, sulfates, sulfenic acids isonitriles, amidines, imides, imidates, nitrones, hydroxylamines, oximes, hydroxamic acids thiohydroxamic acids, allenes, ortho esters, sulfites, enamines, ynamines, ureas, pseudoureas, semicarbazides, carbodiimides, carbamates, imines, azides, azo compounds, azoxy compounds, and nitroso compounds. Reactive functional groups also include those used to prepare bioconjugates, e.g., N-hydroxysuccinimide esters, maleimides, succinimidyl esters (SE), sulfodichlorophenyl (SDP) esters, sulfotetrafluorophenyl (STP) esters, tetrafluorophenyl (TFP) esters, pentafluorophenyl (PFP) esters, nitrilotriacetic acids (NTA), aminodextrans, cyclooctyne-amines and the like. Methods to prepare each of these functional groups are well known in the art and their application to or modification for a particular purpose is within the ability of one of skill in the art (*see*, for example, Sandler and Karo, eds., Organic Functional Group Preparations, Academic Press, San Diego, 1989).

The term "solid support," as used herein, refers to a matrix or medium that is substantially insoluble in liquid phases and capable of binding a molecule or particle of interest. Solid supports suitable for use herein include semi-solid supports and are not limited to a specific type of support. Useful solid supports include solid and semi-solid matrixes, such as aerogels and hydrogels, resins, beads, biochips (including thin film coated biochips), microfluidic chip, a silicon chip, multi-well plates (also referred to as microtitre plates or microplates), membranes, conducting and nonconducting metals, glass (including microscope slides) and magnetic supports. More specific examples of useful solid supports include silica gels, polymeric membranes, particles, derivatized plastic films, glass beads, cotton, plastic beads, alumina gels, polysaccharides such as SEPHAROSE® (GE Healthcare), poly(acrylate), polystyrene, poly(acrylamide), polyol, agarose, agar, cellulose, dextran, starch, FICOLL® (GE Healthcare), heparin, glycogen, amylopectin, mannan, inulin, nitrocellulose, diazocellulose, polyvinylchloride, polypropylene, polyethylene (including poly(ethylene glycol)), nylon, latex bead, magnetic bead, paramagnetic bead, superparamagnetic bead, starch and the like.

### Cell-Tracking Compounds:

In general, for ease of understanding the present disclosure, the cell-tracking compounds and corresponding substituents will first be described in detail, followed by various methods in which the cell-tracking compounds of the present disclosure are useful, which is followed by exemplary methods of use and synthesis of certain cell-tracking compounds that are particularly advantageous for use with the methods provided herein.

The cell-tracking reagent compounds disclosed herein are useful for tracking cell proliferation.

Cell movement and location studies require detectable compounds that are non-toxic to living cells and are available in a range of fluorescent colors to match instrument lasers and filters and to accommodate co-staining with antibodies or other cell analysis compounds. The cell-tracking compounds described herein are useful for monitoring cell movement, location, proliferation, migration, chemotaxis and invasion. The cell-tracking compounds described herein can pass freely through the cell membrane; however, once inside the cell, they are transformed into cell-impermeant reaction products and are well retained in living cells over several generations. The cell-tracking compounds described herein are transferred to daughter cells, but not to adjacent cells in a population. The cell-tracking compounds disclosed herein have the following advantages: they have a high signal:noise ratio by imaging and flow cytometry; they are non-toxic to cells thereby allowing for tracking of multiple cell divisions; they are highly cell permeable; and they are brightly fluorescent at physiological pH.

Advantageously, the cell-tracking compounds described herein can permanently label cells without affecting the cell's morphology or physiology in order to trace generations or divisions both *in vivo* and *in vitro.* The bright, single-peak staining of the cell-tracking compounds described herein enables visualization of multiple generations with long-term signal stability. The cell-tracking compounds described herein are well retained within cells for several days post-stain. The cell-tracking compounds are non-cytotoxic at the indicated concentrations, meaning that they have no known effect on the proliferation ability or biology of the cells. The cell-tracking compounds described herein are non-fluorescent derivatives of reactive fluorescent molecules which enter the cells freely via diffusion. Upon entering the cell, the cell-tracking compounds are acted upon by intracellular enzymes, for example, intracellular esterases, which results in conjugating the cell-tracking compounds to intracellular proteins thereby allowing the conjugated cell-tracking compounds to be retained within the cells. Daughter cells receive approximately half of the label from the parent. Analysis of the level of fluorescence in the cell population by flow cytometry, for example, permits determination of the number of generations through which the cell has progressed since the label was applied (see, FIG. 1A and FIG. 1B).

One illustrative embodiment provides a cell-tracking reagent/cell-tracking compound comprising structural formula (I): 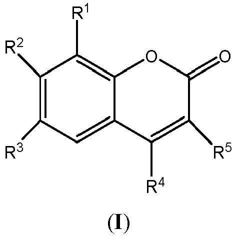 or a pharmaceutically acceptable salt thereof, wherein
R¹ and R³ are each independently hydrogen, halogen, alkyl, or substituted alkyl;
R² is acyl, alkoxy, substituted alkoxy, amino, substituted amino, acylamino, or hydroxyl;
R⁴ is hydrogen, alkyl, or substituted alkyl;
R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
L is a linker;
Rₓ is a reactive group; and
S_{c} is a conjugated substance.

In certain embodiments, R¹ and R³ are each independently hydrogen or halogen. In certain embodiments, R⁴ is alkyl. In certain embodiments, R² is acyl, alkoxy, amino, substituted amino, or hydroxyl. In certain embodiments, R⁵ is -L-Rₓ. In certain embodiments, Rₓ is succinimidyl ester (SE), sulfodichlorophenyl (SDP) ester, sulfotetrafluorophenyl (STP) ester, tetrafluorophenyl (TFP) ester, and pentafluorophenyl (PFP) ester. In certain embodiments, R¹ and R³ are each independently hydrogen or halogen; R⁴ is H or alkyl; R² is acyl, alkoxy, amino, substituted amino, or hydroxyl; and R⁵ is -L-Rₓ, wherein Rₓ is succinimidyl ester (SE), sulfodichlorophenyl (SDP) ester, sulfotetrafluorophenyl (STP) ester, tetrafluorophenyl (TFP) ester, or pentafluorophenyl (PFP) ester.

In certain embodiments, Rₓ is selected from an acrylamide, an activated ester of a carboxylic acid, a carboxylic ester, an acyl azide, an acyl nitrile, an aldehyde, an alkyl halide, an anhydride, an aniline, an amine, an aryl halide, an azide, an aziridine, a boronate, a diazoalkane, a haloacetamide, a haloalkyl, a halotriazine, a hydrazine, an imido ester, an isocyanate, an isothiocyanate, a maleimide, a phosphoramidite, a photoactivatable group, a reactive platinum complex, a silyl halide, a sulfonyl halide, and a thiol. In certain embodiments the reactive group is selected from the group consisting of carboxylic acid, succinimidyl ester of a carboxylic acid, hydrazide, amine and a maleimide. In certain embodiments, Rₓ is a succinimidyl ester.

In certain embodiments, S_{c} is selected from a carrier molecule and a solid support. In certain embodiments, S_{c} is selected from an amino acid, a polymer of amino acids, a peptide, a protein, a neurotoxin, a phallotoxin, a cytokine, a toxin, a protease substrate, a protein kinase substrate, an enzyme, an antibody, an antibody fragment, a lectin, a glycoprotein, a histone, an albumin, a lipoprotein, avidin, streptavidin, protein A, protein G, a phycobiliprotein, a fluorescent protein, a hormone, a growth factor, a nucleic acid base, a nucleoside, a nucleotide, a nucleic acid polymer, a nucleotide analog, a nucleoside analog, a nucleoside triphosphate, a deoxynucleoside triphosphate, a dideoxynucleoside triphosphate, a hapten, a carbohydrate, a polysaccharide, a lipid, an ion-complexing moiety (such as a crown ether), and an organic or inorganic polymer.

Another illustrative embodiment provides a cell-tracking reagent/cell-tracking compound selected from the group consisting of: 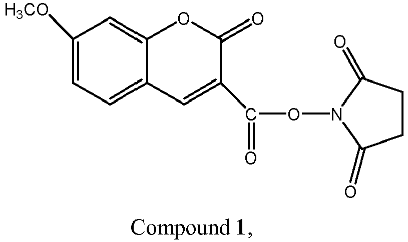 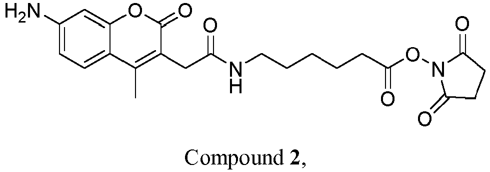 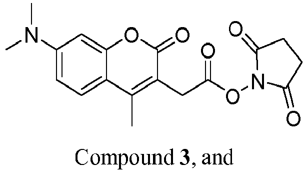 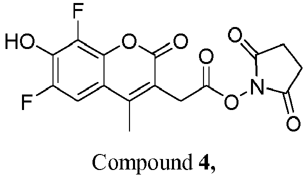 or a pharmaceutically acceptable salt thereof.

### Reactive Groups:

In certain embodiments, the cell-tracker compounds provided herein are chemically reactive, and are substituted by at least one reactive group (Rₓ). The reactive group functions as the site of attachment for another moiety, such as a carrier molecule or a solid support, wherein the reactive group chemically reacts with an appropriate reactive or functional group on the carrier molecule or solid support. Thus, in certain embodiments, the cell-tracker compounds provided herein comprise a fluorophore, a linker, a reactive group moiety and optionally a carrier molecule and/or a solid support.

In certain embodiments, the cell-tracker compounds provided herein further comprise a reactive group which is a member selected from an acrylamide, an activated ester of a carboxylic acid, a carboxylic ester, an acyl azide, an acyl nitrile, an aldehyde, an alkyl halide, an anhydride, an aniline, an amine, an aryl halide, an azide, an aziridine, a boronate, a diazoalkane, a haloacetamide, a haloalkyl, a halotriazine, a hydrazine, an imido ester, an isocyanate, an isothiocyanate, a maleimide, a phosphoramidite, a photoactivatable group, a reactive platinum complex, a silyl halide, a sulfonyl halide, and a thiol. In certain embodiments the reactive group is selected from the group consisting of carboxylic acid, succinimidyl ester of a carboxylic acid, hydrazide, amine and a maleimide. The reactive group may be attached to any appropriate site on the reporter molecule or the fluorophore. Alternatively, if the cell-tracker compounds disclosed herein comprise a carrier molecule or solid support a reactive group may be covalently attached independently to those substituents, allowing for further conjugation to a fluorophore, carrier molecule or solid support.

These reactive groups are synthesized during the formation of the cell-tracker compounds provided herein and carrier molecule- and/or solid support-containing compounds to provide chemically reactive cell-tracker compounds. In this way, cell-tracker compounds incorporating a reactive group may be covalently attached to a wide variety of carrier molecules or solid supports that contain, or are modified to contain, functional groups with suitable reactivity, resulting in chemical attachment of the components. In certain embodiments, the reactive group of the cell-tracker compounds disclosed herein and the functional group of the carrier molecule or solid support comprise electrophiles and nucleophiles that can generate a covalent linkage between them. In certain embodiments, the reactive group comprises a photoactivatable group, which becomes chemically reactive only after illumination with light of an appropriate wavelength. Typically, the conjugation reaction between the reactive group and the carrier molecule or solid support results in one or more atoms of the reactive group being incorporated into a new linkage attaching the cell-tracker compounds disclosed herein to the carrier molecule or solid support. Selected examples of functional groups and linkages are shown in Table 1, where the reaction of an electrophilic group and a nucleophilic group yields a covalent linkage.

**Table 1: Examples of some routes to useful covalent linkages**

| **Electrophilic Group** | **Nucleophilic Group** | **Resulting Covalent Linkage** |
|---|---|---|
| activated esters* | amines/anilines | carboxamides |
| acrylamides | thiols | thioethers |
| acvl azides** | amines/anilines | carboxamides |
| acyl halides | amines/anilines | carboxamides |
| acyl halides | alcohols/phenols | esters |
| acyl nitriles | alcohols/phenols | esters |
| acyl nitriles | amines/anilines | carboxamides |
| aldehydes | amines/anilines | imines |
| aldehydes or ketones | hydrazines | hydrazones |
| aldehydes or ketones | hydroxylamines | oximes |
| alkyl halides | amines/anilines | alkyl amines |
| alkyl halides | carboxylic acids | esters |
| alkyl halides | thiols | thioethers |
| alkyl halides | alcohols/phenols | ethers |
| alkyl sulfonates | thiols | thioethers |
| alkyl sulfonates | carboxylic acids | esters |
| alkyl sulfonates | alcohols/phenols | ethers |
| anhydrides | alcohols/phenols | esters |
| anhydrides | amines/anilines | carboxamides |
| aryl halides | thiols | thiophenols |
| aryl halides | amines | aryl amines |
| aziridines | thiols | thioethers |
| boronates | glycols | boronate esters |
| carbodiimides | carboxylic acids | N-acylureas or anhydrides |
| diazoalkanes | carboxylic acids | esters |
| epoxides | thiols | thioethers |
| haloacetamides | thiols | thioethers |
| haloplatinate | amino | platinum complex |
| haloplatinate | heterocycle | platinum complex |
| haloplatinate | thiol | platinum complex |
| halotriazines | amines/anilines | aminotriazines |
| halotriazines | alcohols/phenols | triazinyl ethers |
| halotriazines | thiols | triazinyl thioethers |
| imido esters | amines/ anilines | amidines |
| isocyanates | amines/ anilines | ureas |
| isocyanates | alcohols/phenols | urethanes |
| isothiocyanates | amines/anilines | thioureas |
| maleimides | thiols | thioethers |
| phosphoramidites | alcohols | phosphite esters |
| silyl halides | alcohols | silyl ethers |
| sulfonate esters | amines/anilines | alkyl amines |
| sulfonate esters | thiols | thioethers |
| sulfonate esters | carboxylic acids | esters |
| sulfonate esters | alcohols | ethers |
| sulfonyl halides | amines/anilines | sulfonamides |
| sulfonyl halides | phenols/alcohols | sulfonate esters |
| | | |
| *Activated esters, as understood in the art, generally have the formula -COΩ, where Ω is a suitable leaving group (*e.g.*, succinimidyloxy (-OC₄H₄O₂), sulfosuccinimidyloxy (-OC₄H₃O₂-SO₃H), -1-oxybenzotriazolyl (-OC₆H₄N₃); or an aryloxy group or aryloxy substituted one or more times by electron withdrawing substituents such as nitro, fluoro, chloro, cyano, or trifluoromethyl, or combinations thereof, used to form activated aryl esters; or a carboxylic acid activated by a carbodiimide to form an anhydride or mixed anhydride -OCOR^{x} or -OCNR^{x}NHR^{y}, where R^{x} and R^{y}, which may be the same or different, are C₁-C₆ alkyl, C₁-C₆ perfluoroalkyl, or C₁-C₆ alkoxy; or cyclohexyl, 3-dimethylaminopropyl, or N-morpholinoethyl). | | |
| **Acyl azides can also rearrange to isocyanates. | | |

The choice of the reactive group used to attach the cell-tracker compounds disclosed herein to the substance to be conjugated typically depends on the reactive or functional group on the substance to be conjugated and the type or length of covalent linkage desired. The types of functional groups typically present on the organic or inorganic substances (biomolecule or non-biomolecule) include, but are not limited to, amines, amides, thiols, alcohols, phenols, aldehydes, ketones, phosphates, imidazoles, hydrazines, hydroxylamines, disubstituted amines, halides, epoxides, silyl halides, carboxylate esters, sulfonate esters, purines, pyrimidines, carboxylic acids, olefinic bonds, or a combination of these groups. A single type of reactive site may be available on the substance (typical for polysaccharides or silica), or a variety of sites may occur (e.g., amines, thiols, alcohols, phenols), as is typical for proteins.

Typically, the reactive group will react with an amine, a thiol, an alcohol, an aldehyde, a ketone, or with silica. Preferably, reactive groups react with an amine or a thiol functional group, or with silica. In certain embodiments, the reactive group is an acrylamide, an activated ester of a carboxylic acid, an acyl azide, an acyl nitrile, an aldehyde, an alkyl halide, a silyl halide, an anhydride, an aniline, an aryl halide, an azide, an aziridine, a boronate, a diazoalkane, a haloacetamide, a halotriazine, a hydrazine (including hydrazides), an imido ester, an isocyanate, an isothiocyanate, a maleimide, a phosphoramidite, a reactive platinum complex, a sulfonyl halide, or a thiol group. As used herein, "reactive platinum complex" refers to chemically reactive platinum complexes such as described in U.S. Patent No. 5,714,327.

In certain embodiments, the cell-tracker compounds disclosed herein comprise at least one reactive group that selectively reacts with an amine group. This amine-reactive group is selected from the group consisting of succinimidyl ester (SE), sulfonyl halide, tetrafluorophenyl (TFP) ester, sulfodichlorophenyl (SDP) ester, sulfotetrafluorophenyl (STP) ester, pentafluorophenyl (PFP) ester, nitrilotriacetic acid (NTA), aminodextran, cyclooctyne-amine and iosothiocyanates. Thus, in certain embodiments, the cell-tracker compounds provided herein form a covalent bond with an amine containing molecule in a sample. In certain embodiments, the cell-tracker compounds provided herein comprise at least one reactive group that selectively reacts with a thiol group. This thiol-reactive group is selected from the group consisting of maleimide, haloalkyl and haloacetamide (including any reactive groups disclosed in U.S. Patent Nos. 5,362,628; 5,352,803 and 5,573,904).

Where the reactive group is an activated ester of a carboxylic acid, such as a succinimidyl ester of a carboxylic acid, a sulfonyl halide, tetrafluorophenyl (TFP) ester, sulfodichlorophenyl (SDP) ester, sulfotetrafluorophenyl (STP) ester, or pentafluorophenyl (PFP) ester, the resulting cell-tracker compound is particularly useful for preparing conjugates of carrier molecules such as proteins, nucleotides, oligonucleotides, or haptens. Where the reactive group is a maleimide, haloalkyl or haloacetamide (including any reactive groups disclosed in U.S. Patent Nos. 5,362,628; 5,352,803 and 5,573,904) the resulting compound is particularly useful for conjugation to thiol-containing substances. Where the reactive group is a hydrazide, the resulting compound is particularly useful for conjugation to periodate-oxidized carbohydrates and glycoproteins, and in addition is an aldehyde-fixable polar tracer for cell microinjection. Where the reactive group is a silyl halide, the resulting compound is particularly useful for conjugation to silica surfaces, particularly where the silica surface is incorporated into a fiber optic probe subsequently used for remote ion detection or quantitation.

In a certain embodiments, the reactive group is a photoactivatable group such that the group is only converted to a reactive species after illumination with an appropriate wavelength. An appropriate wavelength is generally a UV wavelength that is less than 400 nm. This method provides for specific attachment to only the target molecules, either in solution or immobilized on a solid or semi-solid matrix. Photoactivatable reactive groups include, without limitation, benzophenones, aryl azides and diazirines.

Preferably, the reactive group is a photoactivatable group, succinimidyl ester of a carboxylic acid, a haloacetamide, haloalkyl, a hydrazine, an isothiocyanate, a maleimide group, an aliphatic amine, a silyl halide, a cadaverine or a psoralen. More preferably, the reactive group is a succinimidyl ester of a carboxylic acid, a maleimide, an iodoacetamide, or a silyl halide. In certain embodiments, the reactive group is a succinimidyl ester of a carboxylic acid, a sulfonyl halide, a tetrafluorophenyl ester, an iosothiocyanates or a maleimide. In certain embodiments, the reactive group is selected from sulfodichlorophenyl (SDP) ester, sulfotetrafluorophenyl (STP) ester, tetrafluorophenyl (TFP) ester, and a pentafluorophenyl (PFP) ester.

### Carrier Molecules:

In certain embodiments, the cell-tracker compounds provided herein are covalently bound to a carrier molecule. If the cell-tracker compound has a reactive group, then the carrier molecule can alternatively be linked to the cell-tracker compound through the reactive group. The reactive group can contain both a reactive functional moiety and a linker, or only the reactive functional moiety.

A variety of carrier molecules are useful herein. Exemplary carrier molecules include antigens, steroids, vitamins, drugs, haptens, metabolites, toxins, environmental pollutants, amino acids, peptides, proteins, nucleic acids, nucleic acid polymers, carbohydrates, lipids, polymers and bacterial particles.

In certain embodiments, the carrier molecule comprises an amino acid, a peptide, a protein, a polysaccharide, a nucleoside, a nucleotide, an oligonucleotide, a nucleic acid, a hapten, a psoralen, a drug, a hormone, a lipid, a lipid assembly, a synthetic polymer, a polymeric microparticle, a biological cell, a virus and combinations thereof. In certain embodiments, the carrier molecule is selected from a hapten, a nucleotide, an oligonucleotide, a nucleic acid polymer, a protein, a peptide or a polysaccharide. In certain embodiments the carrier molecule is amino acid, a peptide, a protein, a polysaccharide, a nucleoside, a nucleotide, an oligonucleotide, a nucleic acid, a hapten, a psoralen, a drug, a hormone, a lipid, a lipid assembly, a tyramine, a synthetic polymer, a polymeric microparticle, a biological cell, cellular components, an ion chelating moiety, an enzymatic substrate or a virus. In certain embodiments, the carrier molecule is an antibody or fragment thereof, an antigen, an avidin or streptavidin, a biotin, a dextran, an IgG binding protein, a fluorescent protein, agarose, and a non-biological microparticle. In certain embodiments, carrier molecules may comprise a label or a fluorescent dye or quencher.

In certain embodiments, the carrier molecule is an amino acid (including those that are protected or are substituted by phosphates, carbohydrates, or C₁ to C₂₂ carboxylic acids), or a polymer of amino acids such as a peptide or protein. In certain embodiments, the carrier molecule contains at least five amino acids, more preferably 5 to 36 amino acids. Exemplary peptides include, but are not limited to, neuropeptides, cytokines, toxins, protease substrates, and protein kinase substrates. Other exemplary peptides may function as organelle localization peptides, that is, peptides that serve to target the conjugated compound for localization within a particular cellular substructure by cellular transport mechanisms. Preferred protein carrier molecules include enzymes, antibodies, lectins, glycoproteins, histones, albumins, lipoproteins, avidin, streptavidin, protein A, protein G, phycobiliproteins and other fluorescent proteins, hormones, toxins and growth factors. Typically, the protein carrier molecule is an antibody, an antibody fragment, avidin, streptavidin, a toxin, a lectin, a growth factor, bacterial particle or a binding partner for a cell receptor.

In certain embodiments, the carrier molecule comprises a nucleic acid base, nucleoside, nucleotide or a nucleic acid polymer, optionally containing an additional linker or spacer for attachment of a fluorophore or other ligand, such as an alkynyl linkage (U.S. Patent No. 5,047,519), an aminoallyl linkage (U.S. Patent No. 4,711,955) or other linkage. In certain embodiments, the nucleotide carrier molecule is a nucleoside or a deoxynucleoside or a dideoxynucleoside.

Exemplary nucleic acid polymer carrier molecules are single- or multi-stranded, natural or synthetic DNA or RNA oligonucleotides, or DNA/RNA hybrids, or incorporating an unusual linker such as morpholine derivatized phosphates (AntiVirals, Inc., Corvallis OR), or peptide nucleic acids such as *N*-(2-aminoethyl)glycine units, where the nucleic acid contains fewer than 50 nucleotides, more typically fewer than 25 nucleotides.

In certain embodiments, the carrier molecule comprises a carbohydrate or polyol that is typically a polysaccharide, such as dextran, FICOLL® (GE Healthcare), heparin, glycogen, amylopectin, mannan, inulin, starch, agarose and cellulose, or is a polymer such as a poly(ethylene glycol). In certain embodiments, the polysaccharide carrier molecule includes dextran, agarose or FICOLL® (GE Healthcare).

In certain embodiments, the carrier molecule comprises a lipid (typically having 6-25 carbons), including glycolipids, phospholipids, and sphingolipids. In certain embodiments, the carrier molecule comprises a lipid vesicle, such as a liposome, or is a lipoprotein. Some lipophilic substituents are useful for facilitating transport of the conjugated dye into cells or cellular organelles.

In certain embodiments, the carrier molecule is a cell, cellular system, cellular fragment, or subcellular particles, including virus particles, bacterial particles, virus components, biological cells (such as animal cells, plant cells, bacteria, or yeast), or cellular components. Examples of cellular components that are useful as carrier molecules include lysosomes, endosomes, cytoplasm, nuclei, histones, mitochondria, Golgi apparatus, endoplasmic reticulum and vacuoles.

In certain embodiments, the carrier molecule non-covalently associates with organic or inorganic materials. Exemplary embodiments of the carrier molecule that possess a lipophilic substituent may be used to target lipid assemblies such as biological membranes or liposomes by non-covalent incorporation of the cell-tracker compound within the membrane, e.g., for use as probes for membrane structure or for incorporation in liposomes, lipoproteins, films, plastics, lipophilic microspheres or similar materials.

In certain embodiments, the carrier molecule comprises a specific binding pair member wherein the cell-tracker compounds provided herein are conjugated to a specific binding pair member and used to the formation of the bound pair. Alternatively, the presence of the labeled specific binding pair member indicates the location of the complementary member of that specific binding pair; each specific binding pair member having an area on the surface or in a cavity which specifically binds to, and is complementary with, a particular spatial and polar organization of the other. In this instance, the cell-tracker compounds disclosed herein function as a reporter molecule for the specific binding pair. Exemplary binding pairs are set forth in Table 2.

**Table 2: Representative Specific Binding Pairs**

| **Antigen** | **Antibody** |
|---|---|
| biotin | avidin (or streptavidin or anti-biotin) |
| IgG* | protein A or protein G |
| drug | drug receptor |
| folate | folate binding protein |
| toxin | toxin receptor |
| carbohydrate | lectin or carbohydrate receptor |
| peptide | peptide receptor |
| protein | protein receptor |
| enzyme substrate | enzyme |
| DNA (RNA) | cDNA (cRNA)^{†} |
| hormone | hormone receptor |
| ion | chelator |
| | |
| *IgG is an immunoglobulin | |
| ^{†}cDNA and cRNA are the complementary strands used for hybridization | |

### Solid Supports:

In certain embodiments, the cell-tracker compounds disclosed herein are covalently bonded to a solid support. The solid support may be attached to the cell-tracker compounds either through the fluorophore or through a reactive group, if present, or through a carrier molecule, if present. Even if a reactive group and/or a carrier molecule are present, the solid support may be attached through the fluorophore.

Solid supports suitable for use herein are typically substantially insoluble in liquid phases. Solid supports for use herein are not limited to a specific type of support. Rather, a large number of supports are available and are known to one of ordinary skill in the art. Thus, useful solid supports include solid and semi-solid matrixes, such as aerogels and hydrogels, resins, beads, biochips (including thin film coated biochips), microfluidic chip, a silicon chip, multi-well plates (also referred to as microtitre plates or microplates), membranes, conducting and nonconducting metals, glass (including microscope slides) and magnetic supports. More specific examples of useful solid supports include silica gels, polymeric membranes, particles, derivatized plastic films, glass beads, cotton, plastic beads, alumina gels, polysaccharides such as SEPHAROSE® (GE Healthcare), poly(acrylate), polystyrene, poly(acrylamide), polyol, agarose, agar, cellulose, dextran, starch, FICOLL® (GE Healthcare), heparin, glycogen, amylopectin, mannan, inulin, nitrocellulose, diazocellulose, polyvinylchloride, polypropylene, polyethylene (including poly(ethylene glycol)), nylon, latex bead, magnetic bead, paramagnetic bead, superparamagnetic bead, starch and the like.

In certain embodiments, the solid support may include a solid support reactive functional group, including, but not limited to, hydroxyl, carboxyl, amino, thiol, aldehyde, halogen, nitro, cyano, amido, urea, carbonate, carbamate, isocyanate, sulfone, sulfonate, sulfonamide, sulfoxide, *etc.*, for attaching the cell-tracker compounds disclosed herein. Useful reactive groups are disclosed above and are equally applicable to the solid support reactive functional groups herein.

A suitable solid phase support may be selected on the basis of desired end use and suitability for various synthetic protocols. For example, where amide bond formation is desirable to attach the cell-tracker compounds disclosed herein to the solid support, resins generally useful in peptide synthesis may be employed, such as polystyrene (*e.g.*, PAM-resin obtained from Bachem Inc., Peninsula Laboratories, *etc.*), POLYHIPE resin (obtained from Aminotech, Canada), polyamide resin (obtained from Peninsula Laboratories), polystyrene resin grafted with polyethylene glycol (TENTAGEL, Rapp Polymere, Tubingen, Germany), polydimethyl-acrylamide resin (available from Milligen/Biosearch, California), or PEGA beads (obtained from Polymer Laboratories).

### Preparation of Conjugates:

Another illustrative embodiment provides a process for preparing a conjugated cell-tracking compound disclosed herein, the process comprising: reacting a cell-tracking compound disclosed herein as described herein with a substance to be conjugated thereto, thereby resulting in a conjugated substance S*_{C}*.

The cell-tracking compounds disclosed herein that contain a reactive group R*_{X}* are useful to fluorescently label a wide variety of organic substances that contain functional groups with suitable reactivity, resulting in chemical attachment, *i.e.*, conjugation, of the substance (thereby affording a conjugated substance, S*_{C}*) and formation of cell-tracking reagents that are themselves conjugates. Most preferably, but not exclusively, the conjugated substance disclosed herein is an intracellular amino acid, peptide, protein, nucleotide, oligonucleotide, nucleic acid, lipid, phospholipid, lipoprotein, or lipopolysaccharide. The reactive group and functional group are typically an electrophile and a nucleophile, respectively, that can generate a covalent linkage. Alternatively, the reactive group is a photoactivatable group that becomes chemically reactive only after illumination with light of an appropriate wavelength. Selected examples of functional groups and linkages, where the reaction of an electrophilic group and a nucleophilic group yields a covalent linkage, as well as a general discussion of dye-conjugate chemistry, are provided in U.S. Patent No. 5,830,912.

In certain embodiments, conjugates of the cell-tracking compounds disclosed herein are provided. Conjugates of components (carrier molecules or solid supports), e.g., drugs, peptides, toxins, nucleotides, phospholipids, proteins and other organic molecules are prepared by organic synthesis methods using the cell-tracker compounds disclosed herein, are generally prepared by means well recognized in the art (Haugland, MOLECULAR PROBES HANDBOOK, *supra*, (2002)). Preferably, conjugation to form a covalent bond consists of mixing the cell-tracker compounds disclosed herein in a suitable solvent in which both the cell-tracker compound and the substance to be conjugated are soluble. The reaction preferably proceeds spontaneously without added reagents at room temperature or below. For those reactive cell-tracker compounds that are photoactivated, conjugation is facilitated by illumination of the reaction mixture to activate the reactive cell-tracker compound. Chemical modification of water-insoluble substances, so that a desired cell-tracker compound-conjugate may be prepared, is preferably performed in an aprotic solvent such as dimethylformamide, dimethylsulfoxide, acetone, ethyl acetate, toluene, or chloroform. Similar modification of water-soluble materials is readily accomplished through the use of the instant reactive compounds to make them more readily soluble in organic solvents.

Preparation of peptide or protein conjugates typically comprises first dissolving the protein to be conjugated in aqueous buffer at about 1-10 mg/mL at room temperature or below. Bicarbonate buffers (pH about 8.3) are especially suitable for reaction with succinimidyl esters, phosphate buffers (pH about 7.2 - 8) for reaction with thiol-reactive functional groups and carbonate or borate buffers (pH about 9) for reaction with isothiocyanates and dichlorotriazines. The appropriate reactive cell-tracker compound is then dissolved in a nonhydroxylic solvent (usually DMSO or DMF) in an amount sufficient to give a suitable degree of conjugation when added to a solution of the protein to be conjugated. The appropriate amount of cell-tracker compound for any protein or other component is conveniently predetermined by experimentation in which variable amounts of the cell-tracker compound are added to the protein, the conjugate is chromatographically purified to separate unconjugated cell-tracker compound and the cell-tracker compound-protein conjugate is tested in its desired application.

Following addition of the cell-tracker compound to the component solution, the mixture is incubated for a suitable period (typically about 1 hour at room temperature to several hours on ice), the excess cell-tracker compound is removed by gel filtration, dialysis, HPLC, adsorption on an ion exchange or hydrophobic polymer or other suitable means. The cell-tracker compound-conjugate may be used in solution or lyophilized. In this way, suitable conjugates may be prepared from antibodies, antibody fragments, avidins, lectins, enzymes, proteins A and G, cellular proteins, albumins, histones, growth factors, hormones, and other proteins.

Conjugates of polymers, including biopolymers and other higher molecular weight polymers are typically prepared by means well recognized in the art (for example, Brinkley et al., Bioconjugate Chem., 3:2 (1992)). In these embodiments, a single type of reactive site may be available, as is typical for polysaccharides) or multiple types of reactive sites (*e.g.* amines, thiols, alcohols, phenols) may be available, as is typical for proteins. Selectivity of labeling is best obtained by selection of an appropriate reactive cell-tracker compound. For example, modification of thiols with a thiol-selective reagent such as a haloacetamide or maleimide, or modification of amines with an amine-reactive reagent such as an activated ester, acyl azide, isothiocyanate or 3,5-dichloro-2,4,6-triazine. Partial selectivity may also be obtained by careful control of the reaction conditions.

When modifying polymers with the cell-tracker compounds disclosed herein, an excess of cell-tracker compound is typically used, relative to the expected degree of cell-tracker compound substitution. Any residual, unreacted cell-tracker compound or a cell-tracker compound hydrolysis product is typically removed by dialysis, chromatography or precipitation. Presence of residual, unconjugated cell-tracker compound may be detected by thin layer chromatography using a solvent that elutes the cell-tracker compound away from its conjugate. In all cases it is usually preferred that the reagents be kept as concentrated as practical so as to obtain adequate rates of conjugation.

In certain embodiments, the cell-tracker compound-conjugates disclosed herein are associated with an additional substance, that binds either to the fluorophore or the conjugated substance (carrier molecule or solid support) through non-covalent interaction. In another exemplary embodiment, the additional substance is an antibody, an enzyme, a hapten, a lectin, a receptor, an oligonucleotide, a nucleic acid, a liposome, or a polymer. The additional substance is optionally used to probe for the location of the cell-tracker compound-conjugate, for example, as a means of enhancing the signal of the cell-tracker compound-conjugate.

### Cell-Tracking Compositions:

Certain embodiments provide a composition comprising:
a) a compound of the invention, or a pharmaceutically acceptable salt thereof; and
b) a carrier.

In certain embodiments, compositions are provided for tracking cell proliferation, wherein the compositions comprise:
a) one or more of a cell-tracking compound of structural formula (I): 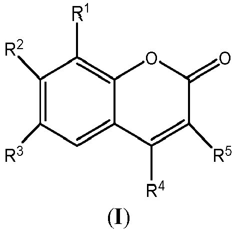 or a pharmaceutically acceptable salt thereof, wherein
   R¹ and R³ are each independently hydrogen, halogen, alkyl, or substituted alkyl;
   R² is acyl, alkoxy, substituted alkoxy, amino, substituted amino, acylamino, or
   hydroxyl;
   R⁴ is hydrogen, alkyl, or substituted alkyl;
   R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
   L is a linker;
   Rₓ is a reactive group; and
   S_{c} is a conjugated substance; and
b) a carrier,
wherein the one or more of the cell-tracker compounds are present in an amount effective to track cell proliferation.

In certain embodiments, R¹ and R³ are each independently hydrogen or halogen. In certain embodiments, R⁴ is alkyl. In certain embodiments, R² is acyl, alkoxy, amino, substituted amino, or hydroxyl. In certain embodiments, R⁵ is -L-Rₓ. In certain embodiments, Rₓ is succinimidyl ester (SE), sulfodichlorophenyl (SDP) ester, sulfotetrafluorophenyl (STP) ester, tetrafluorophenyl (TFP) ester, and pentafluorophenyl (PFP) ester. In certain embodiments, R¹ and R³ are each independently hydrogen or halogen; R⁴ is H or alkyl; R² is acyl, alkoxy, amino, substituted amino, or hydroxyl; and R⁵ is -L-Rₓ; wherein Rₓ is succinimidyl ester (SE), sulfodichlorophenyl (SDP) ester, sulfotetrafluorophenyl (STP) ester, tetrafluorophenyl (TFP) ester, or pentafluorophenyl (PFP) ester.

In certain embodiments, Rₓ is selected from an acrylamide, an activated ester of a carboxylic acid, a carboxylic ester, an acyl azide, an acyl nitrile, an aldehyde, an alkyl halide, an anhydride, an aniline, an amine, an aryl halide, an azide, an aziridine, a boronate, a diazoalkane, a haloacetamide, a haloalkyl, a halotriazine, a hydrazine, an imido ester, an isocyanate, an isothiocyanate, a maleimide, a phosphoramidite, a photoactivatable group, a reactive platinum complex, a silyl halide, a sulfonyl halide, and a thiol. In certain embodiments the reactive group is selected from the group consisting of carboxylic acid, succinimidyl ester of a carboxylic acid, hydrazide, amine and a maleimide. In certain embodiments, Rₓ is a succinimidyl ester.

In certain embodiments, S_{c} is selected from a carrier molecule and a solid support. In certain embodiments, S_{c} is selected from an amino acid, a polymer of amino acids, a peptide, a protein, a neurotoxin, a phallotoxin, a cytokine, a toxin, a protease substrate, a protein kinase substrate, an enzyme, an antibody, an antibody fragment, a lectin, a glycoprotein, a histone, an albumin, a lipoprotein, avidin, streptavidin, protein A, protein G, a phycobiliprotein, a fluorescent protein, a hormone, a growth factor, a nucleic acid base, a nucleoside, a nucleotide, a nucleic acid polymer, a nucleotide analog, a nucleoside analog, a nucleoside triphosphate, a deoxynucleoside triphosphate, a dideoxynucleoside triphosphate, a hapten, a carbohydrate, a polysaccharide, a lipid, an ion-complexing moiety (such as a crown ether), and an organic or inorganic polymer.

In certain embodiments, compositions are provided for tracking cell proliferation, wherein the compositions comprise:
a) one or more of a cell-tracking compound selected from the group consisting of: 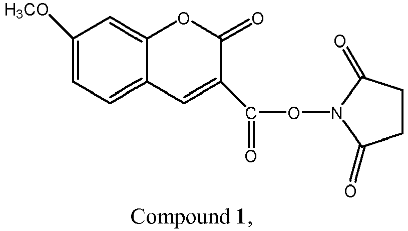 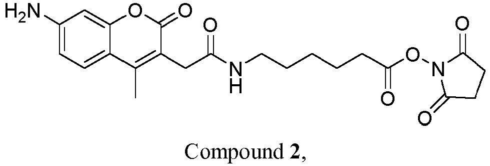 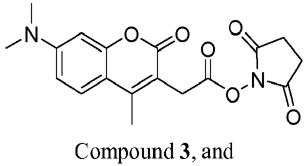 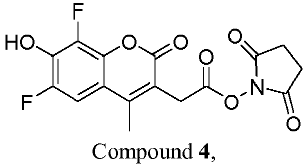 or a pharmaceutically acceptable salt thereof; and
b) a carrier,
wherein the one or more of the cell-tracker compounds are present in an amount effective to track cell proliferation.

Certain embodiments provide a composition comprising:
a) a compound of the invention, or a pharmaceutically acceptable salt thereof; and
b) an analyte.

In certain embodiments, compositions are provided for tracking cell proliferation, wherein the compositions comprise:
(a) one or more of a cell-tracking compound of structural formula (I): 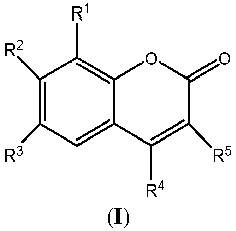 or a pharmaceutically acceptable salt thereof, wherein
   R¹ and R³ are each independently hydrogen, halogen, alkyl, or substituted alkyl;
   R² is acyl, alkoxy, substituted alkoxy, amino, substituted amino, acylamino, or hydroxyl;
   R⁴ is hydrogen, alkyl, or substituted alkyl;
   R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
   L is a linker;
   Rₓ is a reactive group; and
   S_{c} is a conjugated substance; and
(b) an analyte,
wherein the one or more of the cell-tracker compounds are present in an amount effective to track cell proliferation.

In certain embodiments, R¹ and R³ are each independently hydrogen or halogen. In certain embodiments, R⁴ is alkyl. In certain embodiments, R² is acyl, alkoxy, amino, substituted amino, or hydroxyl. In certain embodiments, R⁵ is -L-Rₓ. In certain embodiments, Rₓ is succinimidyl ester (SE), sulfodichlorophenyl (SDP) ester, sulfotetrafluorophenyl (STP) ester, tetrafluorophenyl (TFP) ester, and pentafluorophenyl (PFP) ester. In certain embodiments, R¹ and R³ are each independently hydrogen or halogen; R⁴ is H or alkyl; R² is acyl, alkoxy, amino, substituted amino, or hydroxyl; and R⁵ is -L-Rₓ; wherein Rₓ is succinimidyl ester (SE), sulfodichlorophenyl (SDP) ester, sulfotetrafluorophenyl (STP) ester, tetrafluorophenyl (TFP) ester, or pentafluorophenyl (PFP) ester.

In certain embodiments, Rₓ is selected from an acrylamide, an activated ester of a carboxylic acid, a carboxylic ester, an acyl azide, an acyl nitrile, an aldehyde, an alkyl halide, an anhydride, an aniline, an amine, an aryl halide, an azide, an aziridine, a boronate, a diazoalkane, a haloacetamide, a haloalkyl, a halotriazine, a hydrazine, an imido ester, an isocyanate, an isothiocyanate, a maleimide, a phosphoramidite, a photoactivatable group, a reactive platinum complex, a silyl halide, a sulfonyl halide, and a thiol. In certain embodiments the reactive group is selected from the group consisting of carboxylic acid, succinimidyl ester of a carboxylic acid, hydrazide, amine and a maleimide. In certain embodiments, Rₓ is a succinimidyl ester.

In certain embodiments, S_{c} is selected from a carrier molecule and a solid support. In certain embodiments, S_{c} is selected from an amino acid, a polymer of amino acids, a peptide, a protein, a neurotoxin, a phallotoxin, a cytokine, a toxin, a protease substrate, a protein kinase substrate, an enzyme, an antibody, an antibody fragment, a lectin, a glycoprotein, a histone, an albumin, a lipoprotein, avidin, strcptavidin, protein A, protein G, a phycobiliprotcin, a fluorescent protein, a hormone, a growth factor, a nucleic acid base, a nucleoside, a nucleotide, a nucleic acid polymer, a nucleotide analog, a nucleoside analog, a nucleoside triphosphate, a deoxynucleoside triphosphate, a dideoxynucleoside triphosphate, a hapten, a carbohydrate, a polysaccharide, a lipid, an ion-complexing moiety (such as a crown ether), and an organic or inorganic polymer.

In certain embodiments, compositions are provided for tracking cell proliferation, wherein the compositions comprise:
(a) one or more of a cell-tracking compound selected from the group consisting of: 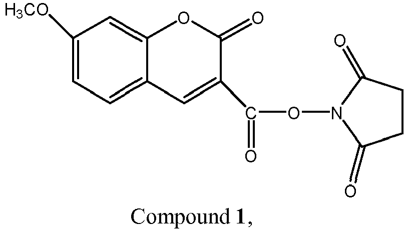 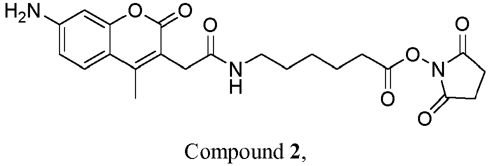 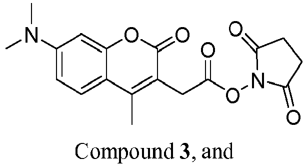 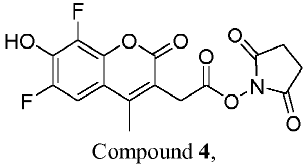 or a pharmaceutically acceptable salt thereof; and
(b) an analyte,
wherein the one or more of the cell-tracker compounds are present in an amount effective to track cell proliferation.

In certain embodiments, the analyte is a cell and the cell-tracker compound is located inside the cell. In certain embodiments, the cell-tracker compound is conjugated to a carrier molecule.

### Methods:

Certain embodiments provide a method for tracking cell proliferation, the method being compatible for use with, for example, flow cytometry and fluorescence microscopy, wherein the method comprises:
a) incubating a mixture of cells with a cell-tracking compound of structural formula (I): 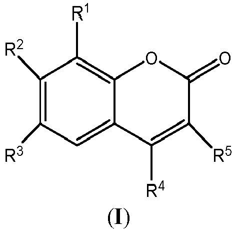 or a pharmaceutically acceptable salt thereof, wherein
   R¹ and R³ are each independently hydrogen, halogen, alkyl, or substituted alkyl;
   R² is acyl, alkoxy, substituted alkoxy, amino, substituted amino, acylamino, or
   hydroxyl;
   R⁴ is hydrogen, alkyl, or substituted alkyl;
   R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
   L is a linker;
   Rₓ is a reactive group; and
   S_{c} is a conjugated substance;
b) providing a stimulus to the mixture to elicit a fluorescent signal; and
c) analyzing the stimulated mixture using flow cytometry.

Certain embodiments provide a method for tracking cell differentiation, the method being compatible for use with, for example, flow cytometry and fluorescence microscopy, wherein the method comprises:
a) incubating a mixture of cells with a cell-tracking compound of structural formula (I): 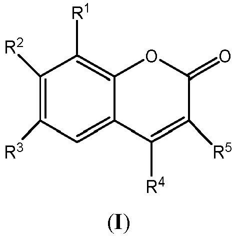 or a pharmaceutically acceptable salt thereof, wherein
   R¹ and R³ are each independently hydrogen, halogen, alkyl, or substituted alkyl;
   R² is acyl, alkoxy, substituted alkoxy, amino, substituted amino, acylamino, or hydroxyl;
   R⁴ is hydrogen, alkyl, or substituted alkyl;
   R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
   L is a linker;
   Rₓ is a reactive group; and
   S_{c} is a conjugated substance;
b) providing a stimulus to the mixture to elicit a fluorescent signal; and
c) analyzing the stimulated mixture using flow cytometry.

In certain embodiments, R¹ and R³ are each independently hydrogen or halogen. In certain embodiments, R⁴ is alkyl. In certain embodiments, R² is acyl, alkoxy, amino, substituted amino, or hydroxyl. In certain embodiments, R⁵ is -L-Rₓ. In certain embodiments, Rₓ is succinimidyl ester (SE), sulfodichlorophenyl (SDP) ester, sulfotetrafluorophenyl (STP) ester, tetrafluorophenyl (TFP) ester, and pentafluorophenyl (PFP) ester. In certain embodiments, R¹ and R³ are each independently hydrogen or halogen; R⁴ is H or alkyl; R² is acyl, alkoxy, amino, substituted amino, or hydroxyl; and R⁵ is -L-Rₓ, wherein Rₓ is succinimidyl ester (SE), sulfodichlorophenyl (SDP) ester, sulfotetrafluorophenyl (STP) ester, tetrafluorophenyl (TFP) ester, or pentafluorophenyl (PFP) ester

In certain embodiments, Rₓ is selected from an acrylamide, an activated ester of a carboxylic acid, a carboxylic ester, an acyl azide, an acyl nitrile, an aldehyde, an alkyl halide, an anhydride, an aniline, an amine, an aryl halide, an azide, an aziridine, a boronate, a diazoalkane, a haloacetamide, a haloalkyl, a halotriazine, a hydrazine, an imido ester, an isocyanate, an isothiocyanate, a maleimide, a phosphoramidite, a photoactivatable group, a reactive platinum complex, a silyl halide, a sulfonyl halide, and a thiol. In certain embodiments the reactive group is selected from the group consisting of carboxylic acid, succinimidyl ester of a carboxylic acid, hydrazide, amine and a maleimide. In certain embodiments, Rₓ is a succinimidyl ester.

In certain embodiments, S_{c} is selected from a carrier molecule and a solid support. In certain embodiments, S_{c} is selected from an amino acid, a polymer of amino acids, a peptide, a protein, a neurotoxin, a phallotoxin, a cytokine, a toxin, a protease substrate, a protein kinase substrate, an enzyme, an antibody, an antibody fragment, a lectin, a glycoprotein, a histone, an albumin, a lipoprotein, avidin, streptavidin, protein A, protein G, a phycobiliprotein, a fluorescent protein, a hormone, a growth factor, a nucleic acid base, a nucleoside, a nucleotide, a nucleic acid polymer, a nucleotide analog, a nucleoside analog, a nucleoside triphosphate, a deoxynucleoside triphosphate, a dideoxynucleoside triphosphate, a hapten, a carbohydrate, a polysaccharide, a lipid, an ion-complexing moiety (such as a crown ether), and an organic or inorganic polymer.

Another embodiment provides a method for tracking cell proliferation, the method being compatible for use with, for example, flow cytometry and fluorescence microscopy, wherein the method comprises:
a) incubating a mixture of cells with a cell-tracking compound selected from the group consisting of: 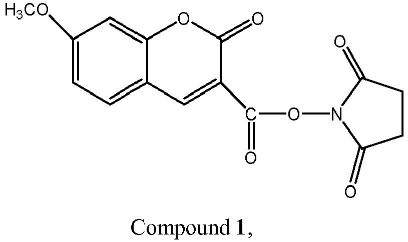 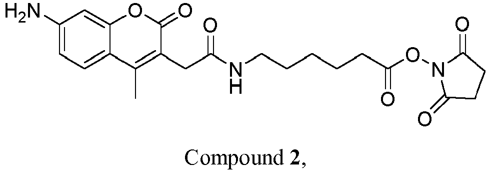 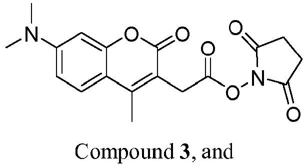 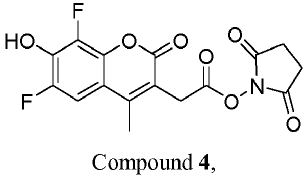 or a pharmaceutically acceptable salt thereof;
b) providing a stimulus to the mixture to elicit a fluorescent signal; and
c) analyzing the stimulated mixture using flow cytometry.

Another embodiment provides a method for tracking cell differentiation, the method being compatible for use with, for example, flow cytometry and fluorescence microscopy, wherein the method comprises:
a) incubating a mixture of cells with a cell-tracking compound selected from the group consisting of: 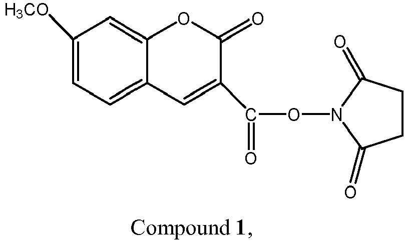 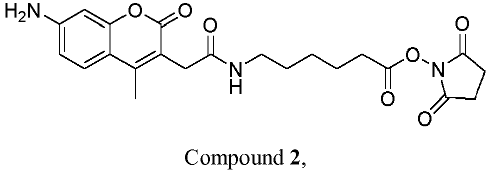 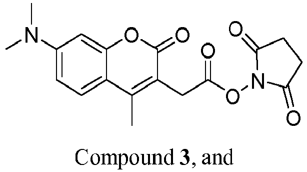 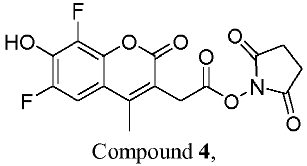 or a pharmaceutically acceptable salt thereof;
b) providing a stimulus to the mixture to elicit a fluorescent signal; and
c) analyzing the stimulated mixture using flow cytometry.

Certain embodiments provide methods for tracking cell proliferation, the methods being compatible for use with, for example, flow cytometry and fluorescence microscopy, wherein the methods comprise:
a) incubating a mixture of cells with a compound of the invention, or a pharmaceutically acceptable salt thereof;
b) providing a stimulus to the mixture to elicit a fluorescent signal; and
c) analyzing the stimulated mixture using flow cytometry.

In one illustrative embodiment of any of the methods described herein, the methods include a second compound excitable at a different wavelength as the first compound. In another illustrative embodiment, the methods include a second compound where the second compound is allophycocyanine (APC), ALEXA FLUOR® 633 fluorescent dye (Thermo Fisher Scientific, Waltham, MA) or Green Fluorescent Protein (GFP).

In another illustrative embodiment of any of the methods described herein, step a) is conducted for approximately 20 minutes. In another illustrative embodiment, step b) and step c) are carried out concurrently. In another illustrative embodiment, step b) and step c) involve flow cytometry.

### Kits:

Another embodiment provides a kit for tracking cell proliferation, the kit being compatible for use with, for example, flow cytometry and fluorescence microscopy, wherein the kit comprises:
a) a cell-tracking compound of structural formula (I): 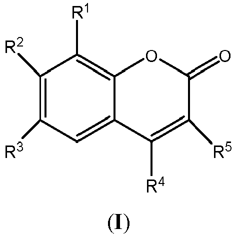 or a pharmaceutically acceptable salt thereof, wherein
   R¹ and R³ are each independently hydrogen, halogen, alkyl, or substituted alkyl;
   R² is acyl, alkoxy, substituted alkoxy, amino, substituted amino, acylamino, or hydroxyl;
   R⁴ is hydrogen, alkyl, or substituted alkyl;
   R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
   L is a linker;
   Rₓ is a reactive group; and
   S_{c} is a conjugated substance;
b) an organic solvent; and
c) a desiccant.

Another embodiment provides a kit for tracking cell differentiation, the kit being compatible for use with, for example, flow cytometry and fluorescence microscopy, wherein the kit comprises:
a) a cell-tracking compound of structural formula (I): 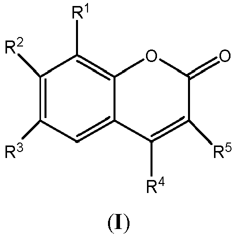 or a pharmaceutically acceptable salt thereof, wherein
   R¹ and R³ are each independently hydrogen, halogen, alkyl, or substituted alkyl;
   R² is acyl, alkoxy, substituted alkoxy, amino, substituted amino, acylamino, or hydroxyl;
   R⁴ is hydrogen, alkyl, or substituted alkyl;
   R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
   L is a linker;
   Rₓ is a reactive group; and
   S_{c} is a conjugated substance;
b) an organic solvent; and
c) a desiccant.

In certain embodiments, R¹ and R³ are each independently hydrogen or halogen. In certain embodiments, R⁴ is alkyl. In certain embodiments, R² is acyl, alkoxy, amino, substituted amino, or hydroxyl. In certain embodiments, R⁵ is -L-Rₓ. In certain embodiments, Rₓ is succinimidyl ester (SE), sulfodichlorophenyl (SDP) ester, sulfotetrafluorophenyl (STP) ester, tetrafluorophenyl (TFP) ester, and pentafluorophenyl (PFP) ester. In certain embodiments, R¹ and R³ are each independently hydrogen or halogen; R⁴ is H or alkyl; R² is acyl, alkoxy, amino, substituted amino, or hydroxyl; and R⁵ is -L-Rₓ, wherein Rₓ is succinimidyl ester (SE), sulfodichlorophenyl (SDP) ester, sulfotetrafluorophenyl (STP) ester, tetrafluorophenyl (TFP) ester, or pentafluorophenyl (PFP) ester.

In certain embodiments, Rₓ is selected from an acrylamide, an activated ester of a carboxylic acid, a carboxylic ester, an acyl azide, an acyl nitrile, an aldehyde, an alkyl halide, an anhydride, an aniline, an amine, an aryl halide, an azide, an aziridine, a boronate, a diazoalkane, a haloacetamide, a haloalkyl, a halotriazine, a hydrazine, an imido ester, an isocyanate, an isothiocyanate, a maleimide, a phosphoramidite, a photoactivatable group, a reactive platinum complex, a silyl halide, a sulfonyl halide, and a thiol. In certain embodiments the reactive group is selected from the group consisting of carboxylic acid, succinimidyl ester of a carboxylic acid, hydrazide, amine and a maleimide. In certain embodiments, Rₓ is a succinimidyl ester.

In certain embodiments, S_{c} is selected from a carrier molecule and a solid support. In certain embodiments, S_{c} is selected from an amino acid, a polymer of amino acids, a peptide, a protein, a neurotoxin, a phallotoxin, a cytokine, a toxin, a protease substrate, a protein kinase substrate, an enzyme, an antibody, an antibody fragment, a lectin, a glycoprotein, a histone, an albumin, a lipoprotein, avidin, streptavidin, protein A, protein G, a phycobiliprotein, a fluorescent protein, a hormone, a growth factor, a nucleic acid base, a nucleoside, a nucleotide, a nucleic acid polymer, a nucleotide analog, a nucleoside analog, a nucleoside triphosphate, a deoxynucleoside triphosphate, a dideoxynucleoside triphosphate, a hapten, a carbohydrate, a polysaccharide, a lipid, an ion-complexing moiety (such as a crown ether), and an organic or inorganic polymer.

Another embodiment provides a kit for tracking cell proliferation, the kit being compatible for use with, for example, flow cytometry and fluorescence microscopy, wherein the kit comprises:
a) a cell-tracking compound selected from the group consisting of: 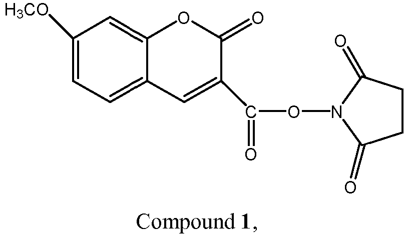 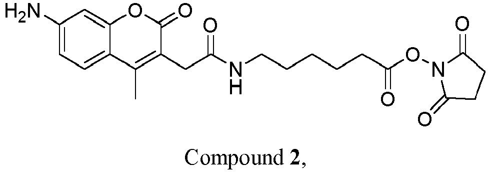 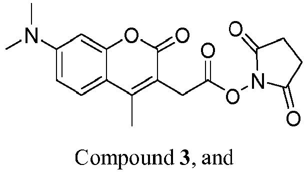 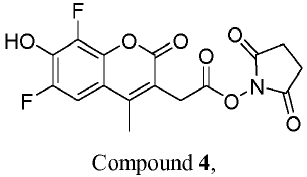 or a pharmaceutically acceptable salt thereof;
b) an organic solvent; and
c) a desiccant.

Another embodiment provides a kit for tracking cell differentiation, the kit being compatible for use with, for example, flow cytometry and fluorescence microscopy, wherein the kit comprises:
a) a cell-tracking compound selected from the group consisting of: 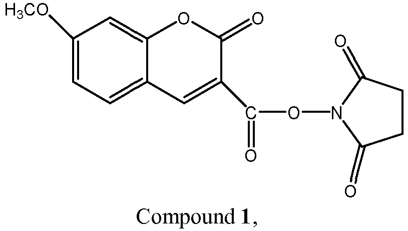 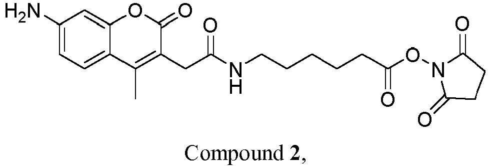 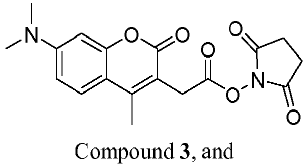 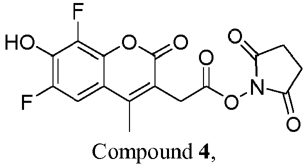 or a pharmaceutically acceptable salt thereof;
b) an organic solvent; and
c) a desiccant.

In another illustrative embodiment of any of the kits provided herein, Rₓ is a succinimidyl ester. In another illustrative embodiment, the organic solvent is DMSO.

In certain embodiments, kits are provided for tracking cell proliferation, the kits comprising:
(a) one or more of the cell-tracking compounds, or a pharmaceutically acceptable salt thereof described herein;
(b) one or more containers; and optionally
(c) instructions for tracking cell proliferation according to a method disclosed herein.

In certain embodiments, kits are provided for tracking cell proliferation, the kits comprising:
(a) one or more compounds of the invention, or a pharmaceutically acceptable salt thereof;
(b) one or more containers; and optionally
(c) instructions for tracking cell proliferation according to a method disclosed herein.

In certain embodiments, the kits further comprise one or more of the following: a buffering agent, a purification medium, a vial comprising the sample, or an organic solvent.

As used herein, the term "kit" refers to a packaged set of related components, typically one or more cell-tracking compounds or compositions. In certain embodiments, the kits disclosed herein comprise one or more of the cell-tracking compounds described herein, one or more carriers suitable for *in vitro* or *in vivo* applications, and one or more containers in which to store the one or more cell-tracking compounds and/or one or more carriers, such as solvents, buffers, stabilizers, pH adjusting agents, *etc.* The kit optionally contains instructions for how to prepare the one or more cell-tracking compounds or how to prepare a composition containing the one or more cell-tracking compounds, and how to administer the cell-tracking compound or composition containing the cell-tracking compound. In certain embodiments, the kit comprises instructions for performing an assay that tracks cellular proliferation. In certain embodiments, the assay is an *in vitro* assay. In certain embodiments, the assay is an *in vivo* assay. The kit may further comprise one or more pieces of equipment to administer the cell-tracker compound, or composition containing the cell-tracker compound including, but not limited to, syringes, pipettes, pipette bulbs, spatulas, vials, syringe needles, and various combinations thereof.

In certain embodiments, the kits provided herein comprise indicator solutions or indicator "dipsticks", blotters, culture media, cuvettes, and the like. In certain embodiments, the kits provided herein comprise indicator cartridges (where a kit component is bound to a solid support) for use in an automated detector. In certain embodiments, the kits provided herein further comprise molecular weight markers, wherein said markers are selected from phosphorylated and non-phosphorylatcd polypeptides, calcium-binding and non-calcium binding polypeptides, sulfonated and non-sulfonated polypeptides, and sialylated and non-sialylated polypeptides. In certain embodiments, the kits provided herein further comprise a member selected from a fixing solution, a detection reagent, a standard, a wash solution, and combinations thereof.

In certain embodiments, kits for tracking cell proliferation are provided, the kits comprising:
(a) one or more of the compositions, or a pharmaceutically acceptable salt thereof described herein;
(b) one or more containers; and optionally
(c) instructions for tracking cell proliferation according to a method disclosed herein.

In certain embodiments, kits for tracking cell proliferation are provided, the kits comprising:
(a) one or more of the compositions of the invention, or a pharmaceutically acceptable salt thereof;
(b) one or more containers; and optionally
(c) instructions for tracking cell proliferation according to a method disclosed herein.

A detailed description of the present teachings having been provided above, the following examples are given for the purpose of illustrating the present teachings and shall not be construed as being a limitation on the scope of the disclosure or claims.

### EXAMPLES

### Example 1: Generational studies of cells incubated with Compound 1:

### I. Separation of mononuclear cells from whole blood:

20 mL whole blood were added to 20 mL DPBS and mixed well. 15 mL FICOLL-PAQUE® PLUS (GE Healthcare) were added to each of two 50 mL centrifuge tubes. 20 mL of the diluted whole blood was carefully layered on top. The tube containing the whole blood was centrifuged for 30 min at 400 x *g* and the peripheral blood mononuclear cell (PBMC) layer was carefully removed. The cells were resuspended in 25 mL DPBS in a 50 mL centrifuge tube and centrifuged for 5 min at 400 x *g.* The supernatant was decanted and the cells were resuspended in 10 mL DPBS. Cells were counted on a COUNTESS Automated Cell Counter (Thermo Fisher Scientific) with the concentration adjusted to 1 × 10⁶ cells/mL in DPBS.

### II. Cell staining:

20 µL anhydrous DMSO was added to a vial of dry Compound **1** (64 µg) at room temperature. 1 mL of unstained cells was reserved as a control. Cells were labeled with 1 µL of 10 mM Compound 1, per milliliter of cell suspension. The tubes were immediately vortexed for 30 sec and the cells were incubated for 20 min at room temperature, protected from light. Five times the volume of stained cells of prepared OPTMIZER CTS T-Cell Expansion SFM medium (Thermo Fisher Scientific) was added and incubated for 5 min at room temperature, protected from light. The cells were pelleted by centrifuging 5 min at 400 x *g* and the supernatant was decanted. The cell pellet was resuspended in 2 mL DPBS + 10% FBS. The cells were pelleted by centrifuging 5 min at 400 x *g* and then decanting the supernatant. The cell pellet was resuspended in 10 mL of prepared OPTMIZER CTS T-Cell Expansion SFM medium. Aliquots of the stained cells were distributed into a 6-well or 24-well culture plate (at least 1 mL per well).

### III. Stimulating and culturing T lymphocytes for proliferation analysis with DYNABEADS® (Invitrogen Dynal AS) Human T-Activator CD3/CD28:

Each 1 mL aliquot of cells was stimulated by adding 100 ng IL-2 (1 µL of 0.1 mg/mL solution. DYNABEADS® (Invitrogen Dynal AS) were resuspended by brief vortexing. The desired volume of DYNABEADS® (Invitrogen Dynal AS) was transferred to a 12 × 75 mm flow cytometry tube (50 µL of beads for each milliliter of cells to be stimulated). The same volume of DPBS, or at least 1 mL, was added and mixed. The tube was placed in the DYNAMAG-15 magnet (Thermo Fisher Scientific) for 1 min. The solution was decanted from the tube, leaving the beads bound to the magnet. The tube was removed from the DYNAMAG-15 magnet and the DYNABEADS® (Invitrogen Dynal AS) were resuspended in the same volume of DPBS as the initial volume of DYNABEADS® (Invitrogen Dynal AS). 50 µL CD3/CD28 DYNABEADS® (Invitrogen Dynal AS) Human T-Activator CD3/CD28 T cell expander beads (2 beads per cell) were added. Cells were incubated at 37°C and 5% CO₂.

### IV. Maintenance of cells in culture:

Every other day while in culture: 1 ml of OPTMIZER CTS T-Cell Expansion SFM medium was added to the cells. Each sample was triturated with a pipet to break up clumps.

### V. Removing the cells from culture after Day 6 (see FIG 2A) or 7 (see FIG. 2B):

2 mL of each sample were removed by first mixing sample with a pipet then transferring them to their own tubes. The remaining samples were left in plate to continue to proliferate. The tube was placed in the DYNAMAG-15 magnet for 1 min. While still in the magnet, the supernatant was removed and transferred to a new tube. The cells were pelleted by centrifuging 5 min at 400 x *g,* then pouring off the supernatant. The cell pellet was resuspended in 5 mL DPBS + 10% FBS. The cells were pelleted by centrifuging 5 min at 400 x *g* and the supernatant was decanted. Cells were counted on a COUNTESS Automated Cell Counter with the concentration adjusted to 1 × 10⁶ cells/mL in DPBS. Samples were placed on ice.

### VI. Single-color compensation control setup:

Spectral overlap between the fluorescent labels in this experiment is minimal but must be corrected by compensation using single-color controls. The first of these controls was prepared using compensation beads. One drop of antibody capture beads from the ABC AntiMouse Bead Kit (Thermo Fisher Scientific) was added to a 1.5 mL microcentrifuge tube or 12 × 75 mm flow cytometry tube. The beads were labeled with 5 µL of mouse anti-human direct conjugate and incubated for 20 min at room temperature, protected from light. Cells were centrifuged for 5 min at 400 x *g*, the supernatant was decanted and the cells were resuspended in 1 mL DPBS. 1 mL of heat-killed or fixed PBMCs were added (1 × 10⁶ cells/mL in DPBS) to a 1.5 mL microcentrifuge tube or 12 × 75 mm flow cytometry tube. The cells were labeled with 1 µL SYTOX AADVANCED (Thermo Fisher Scientific) Dead Cell Stain with a final concentration of 1 µM and was incubated for 5 min at room temperature. 1 mL of unstimulated PBMCs or cells labeled with Compound 1 was added to a 1.5 mL microcentrifuge tube or 12 × 75 mm tube. 1 ml cells were labeled with 5 µL CD4 Mouse anti-human and incubated for 15 min at room temperature. Cells were washed two times in 5 ml PBS and resuspended at 1 × 10⁶ cells/mL in DPBS.

### VII. Setting PMT voltages, threshold, and compensation:

The instrument's performance was verified with ATTUNE Performance Tracking Beads (Thermo Fisher Scientific).

### VIII. Discussion:

As is shown in FIG. 2A, cell proliferation was followed for 7 days using Compound **1.** Human peripheral blood mononuclear cells (PBMCs) were harvested and stained with Compound **1** prior to stimulation with Interleukin-2 (Cat. no. PHC0027) and CD3/CD28 DYNABEADS (Cat no. 111-41D). The unstimulated parent generation is indicated as the peak furthest to the right and the discrete peaks to the left of the unstimulated peak represent successive generations of live, CD4 positive cells. Acquisition was performed on the BD LSRII flow cytometer using a 355nm laser and 450/50 bandpass emission filter. Analysis was completed using the ATTUNE Acoustic Focusing Cytometer software.

As is shown in FIG. 2B, cell proliferation was followed for 6 days using Compound **1.** Human peripheral blood mononuclear cells (PBMCs) were harvested and stained with Compound **1** prior to stimulation with Interleukin-2 (Cat. no. PHC0027) and CD3/CD28 DYNABEADS (Cat no. 111-41D). The unstimulated parent generation is indicated as the peak furthest to the right and the discrete peaks to the left of the unstimulated peak represent successive generations of live, CD4 positive cells. Acquisition was performed on the BD LSRII flow cytometer using a 355nm laser and 450/50 bandpass emission filter. Analysis was completed using the ATTUNE Acoustic Focusing Cytometer software.

## Claims

1. A method for tracking cell proliferation, the method comprising the steps of:
a) incubating a mixture of cells with a compound of structural formula (I): 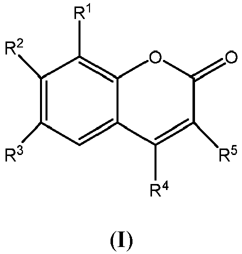 or a pharmaceutically acceptable salt thereof, wherein
R¹ and R³ are each independently hydrogen, halogen, alkyl, or substituted alkyl;
R² is acyl, alkoxy, substituted alkoxy, amino, substituted amino, acylamino, or hydroxyl;
R⁴ is hydrogen, alkyl, or substituted alkyl;
R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
L is a linker;
Rₓ is a reactive group; and
S_{c} is a conjugated substance;
b) providing a stimulus to the mixture to elicit a fluorescent signal; and
c) analyzing the stimulated mixture using flow cytometry.

2. The method according to claim 1, wherein
R¹ and R³ are each independently hydrogen or halogen;
R² is acyl, alkoxy, amino, substituted amino, or hydroxyl;
R⁴ is H or alkyl; and
R⁵ is -L-Rₓ,
wherein Rₓ is succinimidyl ester (SE), sulfodichlorophenyl (SDP) ester, sulfotetrafluorophenyl (STP) ester, tetrafluorophenyl (TFP) ester, or pentafluorophenyl (PFP) ester.

3. The method according to claim 1, wherein the compound of structural formula **(I)** is selected from the group consisting of: 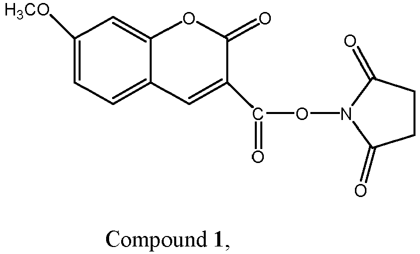 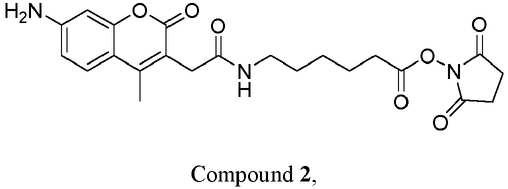 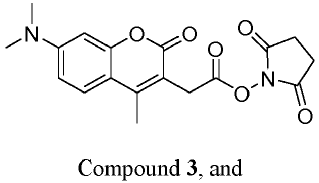 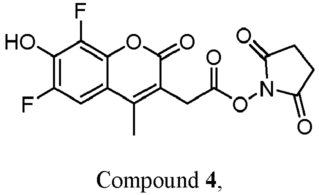 or a pharmaceutically acceptable salt thereof.

4. The method according to any one of claims 1-3, further comprising a second compound excitable at a different wavelength than the cell-tracking compound.

5. The method according to claim 1 or 2, wherein Rₓ is an acrylamide, a carboxylic acid, an activated ester of a carboxylic acid, an acyl azide, an acyl halide, hydroxy, an aldehyde, an alkyl halide, a sulfonate, an amine, an anhydride, an aniline, an aryl halide, an azide, an aziridine, a boronate, a carbodiimide, a diazoalkane, an epoxide, a glycol, a haloacetamide, a halotriazine, a hydrazine, a hydroxylamine, an imido ester, an isothiocyanate, a ketone, a maleimide, a sulfonyl halide, or a thiol group; or
wherein Rₓ is a carboxylic acid, an activated ester of a carboxylic acid, an amine, a maleimide, an iodoacetamide, an isothiocyanate, or a halomethyl; or
wherein Rₓ is selected from a succinimidyl ester (SE), sulfodichlorophenyl (SDP) ester, sulfotetrafluorophenyl (STP) ester, tetrafluorophenyl (TFP) ester, and pentafluorophenyl (PFP) ester.

6. The method according to any one of the preceding claims, wherein step a) is conducted for approximately 20 minutes.

7. The method according to any one of the preceding claims, wherein step b) and step c) are carried out concurrently.

8. Use of a kit for tracking cell proliferation in a method according to any one of claims 1 to 7, the kit comprising:
a) a compound of structural formula (I): 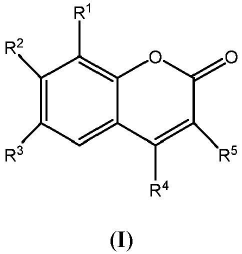 or a pharmaceutically acceptable salt thereof, wherein
R¹ and R³ are each independently hydrogen, halogen, alkyl, or substituted alkyl;
R² is alkoxy, substituted alkoxy, amino, substituted amino, acylamino, or hydroxyl;
R⁴ is hydrogen, alkyl, or substituted alkyl;
R⁵ is alkyl, substituted alkyl, acyloxy, aminocarbonyl, aminocarbonylamino, aminocarbonyloxy, carbonyl, carboxyl, carboxylalkyl, carboxylester, (carboxylester)amino, -L-Rₓ, or -L-S_{c};
L is a linker;
Rₓ is a reactive group; and
S_{c} is a conjugated substance;
b) an organic solvent; and
c) a desiccant.

9. The use of a kit according to claim 8, wherein
R¹ and R³ are each independently hydrogen or halogen;
R² is acyl, alkoxy, amino, substituted amino, or hydroxyl;
R⁴ is H or alkyl; and
R⁵ is -L-Rₓ,
wherein Rₓ is succinimidyl ester (SE), sulfodichlorophenyl (SDP) ester, sulfotetrafluorophenyl (STP) ester, tetrafluorophenyl (TFP) ester, or pentafluorophenyl (PFP) ester.

10. The use of a kit according to claim 8, wherein the compound of structural formula (I) is selected from the group consisting of: 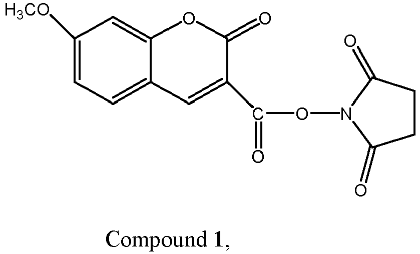 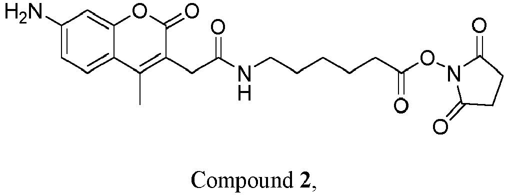 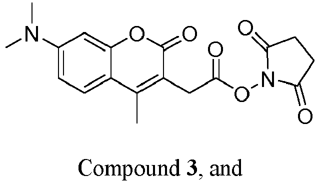 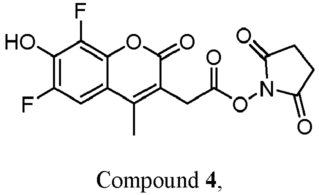 or a pharmaceutically acceptable salt thereof.

11. The use of a kit according to claim 8 or 9, wherein R*_{X}* is an acrylamide, a carboxylic acid, an activated ester of a carboxylic acid, an acyl azide, an acyl halide, hydroxy, an aldehyde, an alkyl halide, a sulfonate, an amine, an anhydride, an aniline, an aryl halide, an azide, an aziridine, a boronate, a carbodiimide, a diazoalkane, an epoxide, a glycol, a haloacetamide, a halotriazine, a hydrazine, a hydroxylamine, an imido ester, an isothiocyanate, a ketone, a maleimide, a sulfonyl halide, or a thiol group; or
wherein R*_{X}* is a carboxylic acid, an activated ester of a carboxylic acid, an amine, a maleimide, an iodoacetamide, an isothiocyanate, or a halomethyl; or
wherein R*_{X}* is selected from a succinimidyl ester (SE), sulfodichlorophenyl (SDP) ester, sulfotetrafluorophenyl (STP) ester, tetrafluorophenyl (TFP) ester, and pentafluorophenyl (PFP) ester.

12. The use of a kit according to any one of claims 8-11, wherein the organic solvent is DMSO.

13. Use of a composition for tracking cell proliferation in a method according to any one of claims 1 to 7, the composition comprising:
a) one or more of the cell-tracker compounds according to any one of claims 1 to 3; and
b) a carrier or an analyte,
wherein the one or more of the cell-tracker compounds are present in an amount effective to track cell proliferation.

14. The use of a composition according to claim 13, wherein the cell-tracker compound is conjugated to a carrier molecule.

15. The use of a composition according to claim 13, wherein the analyte is a cell and the cell-tracker compound is located inside the cell.

## Patentansprüche

1. Verfahren zum Verfolgen einer Zellproliferation, wobei das Verfahren die folgenden Schritte umfasst:
a) Inkubieren einer Mischung von Zellen mit einer Verbindung der Strukturformel (I): 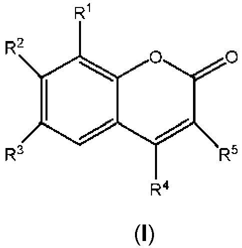 oder einem pharmazeutisch unbedenklichen Salz davon, wobei
R¹ und R³ jeweils unabhängig Wasserstoff, Halogen, Alkyl oder substituiertes Alkyl sind;
R² Acyl, Alkoxy, substituiertes Alkoxy, Amino, substituiertes Amino, Acylamino oder Hydroxyl ist;
R⁴ Wasserstoff, Alkyl oder substituiertes Alkyl ist;
R⁵ Alkyl, substituiertes Alkyl, Acyloxy, Aminocarbonyl, Aminocarbonylamino, Aminocarbonyloxy, Carbonyl, Carboxyl, Carboxylalkyl, Carboxylester, (Carboxylester)amino, -L-Rₓ oder -L-S_{c} ist,
L ein Linker ist;
Rₓ eine reaktive Gruppe ist; und
S_{c} eine konjugierte Substanz ist;
b) Bereitstellen eines Stimulus an die Mischung, um ein Fluoreszenzsignal hervorzurufen; und
c) Analysieren der stimulierten Mischung unter Verwendung einer Durchflusszytometrie.

2. Verfahren nach Anspruch 1, wobei
R¹ und R³ jeweils unabhängig Wasserstoff oder Halogen sind;
R² Acyl, Alkoxy, Amino, substituiertes Amino oder Hydroxyl ist;
R⁴ H oder Alkyl ist; und
R⁵ -L-Rₓ ist,
wobei Rₓ Succinimidylester (SE), Sulfodichlorphenyl(SDP)-Ester, Sulfotetrafluorphenyl(STP)-Ester, Tetrafluorphenyl(TFP)-Ester oder Pentafluorphenyl(PFP)-Ester ist.

3. Verfahren nach Anspruch 1, wobei die Verbindung der Strukturformel (I) aus der Gruppe ausgewählt ist, die aus Folgendem besteht: 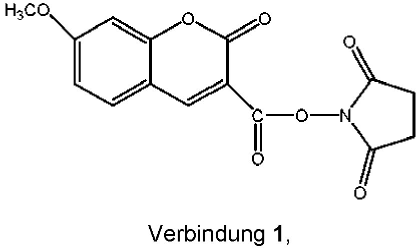 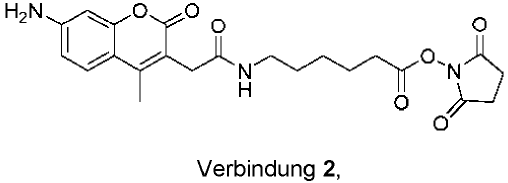 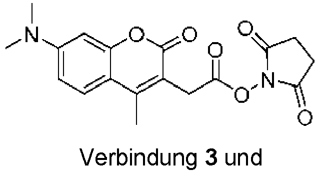 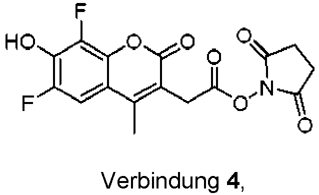 oder einem pharmazeutisch unbedenklichen Salz davon.

4. Verfahren nach einem der Ansprüche 1-3, das ferner eine zweite Verbindung umfasst, die bei einer anderen Wellenlänge als die Zellverfolgungsverbindung erregbar ist.

5. Verfahren nach Anspruch 1 oder 2, wobei Rₓ ein Acrylamid, eine Carbonsäure, ein aktivierter Ester einer Carbonsäure, ein Acylazid, ein Acylhalogenid, Hydroxy, ein Aldehyd, ein Alkylhalogenid, ein Sulfonat, ein Amin, ein Anhydrid, ein Anilin, ein Arylhalogenid, ein Azid, ein Aziridin, ein Boronat, ein Carbodiimid, ein Diazoalkan, ein Epoxid, ein Glycol, ein Halogenacetamid, ein Halotriazin, ein Hydrazin, ein Hydroxylamin, ein Imidoester, ein Isothiocyanat ein Keton, ein Maleimid, ein Sulfonylhalogenid oder eine Thiolgruppe ist; oder
wobei Rₓ eine Carbonsäure, ein aktivierter Ester einer Carbonsäure, ein Amin, ein Maleimid, ein lodacetamid, ein Isothiocyanat oder ein Halogenmethyl ist; oder
wobei Rₓ aus einem Succinimidylester (SE), Sulfodichlorphenyl(SDP)-Ester, Sulfotetrafluorphenyl(STP)-Ester, Tetrafluorphenyl(TFP)-Ester und Pentafluorphenyl(PFP)-Ester ausgewählt ist.

6. Verfahren nach einem der vorhergehenden Ansprüche, wobei Schritt a) ungefähr 20 Minuten lang durchgeführt wird.

7. Verfahren nach einem der vorhergehenden Ansprüche, wobei Schritt b) und Schritt c) gleichzeitig ausgeführt werden.

8. Verwendung eines Kits zum Verfolgen der Zellproliferation in einem Verfahren nach einem der Ansprüche 1 bis 7, wobei das Kit Folgendes umfasst:
a) eine Verbindung der Strukturformel (I): 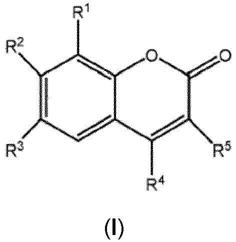 oder ein pharmazeutisch unbedenkliches Salz davon, wobei
R¹ und R³ jeweils unabhängig Wasserstoff, Halogen, Alkyl oder substituiertes Alkyl sind;
R² Alkoxy, substituiertes Alkoxy, Amino, substituiertes Amino, Acylamino oder Hydroxyl ist;
R⁴ Wasserstoff, Alkyl oder substituiertes Alkyl ist;
R⁵ Alkyl, substituiertes Alkyl, Acyloxy, Aminocarbonyl, Aminocarbonylamino, Aminocarbonyloxy, Carbonyl, Carboxyl, Carboxylalkyl, Carboxylester, (Carboxylester)amino, -L-Rₓ oder -L-S_{c} ist,
L ein Linker ist;
Rₓ eine reaktive Gruppe ist; und
S_{c} eine konjugierte Substanz ist;
b) ein organisches Lösungsmittel; und
c) ein Trockenmittel.

9. Verwendung eines Kits nach Anspruch 8, wobei
R¹ und R³ jeweils unabhängig Wasserstoff oder Halogen sind;
R² Acyl, Alkoxy, Amino, substituiertes Amino oder Hydroxyl ist;
R⁴ H oder Alkyl ist; und
R⁵ -L-Rₓ ist,
wobei Rₓ Succinimidylester (SE), Sulfodichlorphenyl(SDP)-Ester, Sulfotetrafluorphenyl(STP)-Ester, Tetrafluorphenyl(TFP)-Ester oder Pentafluorphenyl(PFP)-Ester ist.

10. Verwendung eines Kits nach Anspruch 8, wobei die Verbindung der Strukturformel **(I)** aus der Gruppe ausgewählt ist, die aus Folgendem besteht: 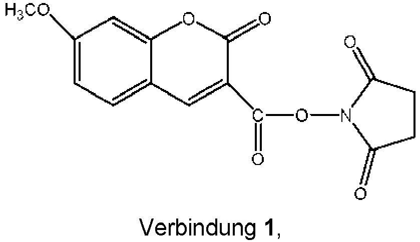 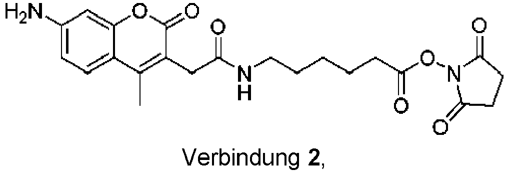 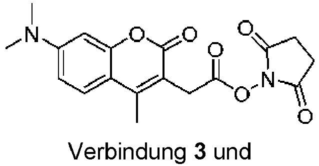 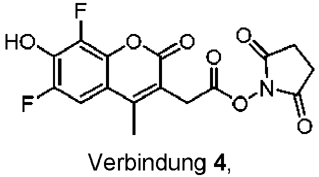 oder einem pharmazeutisch unbedenklichen Salz davon.

11. Verwendung eines Kits nach Anspruch 8 oder 9, wobei R*_{X}* ein Acrylamid, eine Carbonsäure, ein aktivierter Ester einer Carbonsäure, ein Acylazid, ein Acylhalogenid, Hydroxy, ein Aldehyd, ein Alkylhalogenid, ein Sulfonat, ein Amin, ein Anhydrid, ein Anilin, ein Arylhalogenid, ein Azid, ein Aziridin, ein Boronat, ein Carbodiimid, ein Diazoalkan, ein Epoxid, ein Glycol, ein Halogenacetamid, ein Halotriazin, ein Hydrazin, ein Hydroxylamin, ein Imidoester ein Isothiocyanat, ein Keton, ein Maleimid, ein Sulfonylhalogenid oder eine Thiolgruppe ist; oder
wobei R*_{X}* eine Carbonsäure, ein aktivierter Ester einer Carbonsäure, ein Amin, ein Maleimid, ein lodacetamid, ein Isothiocyanat oder ein Halogenmethyl ist; oder
wobei R*_{X}* aus einem Succinimidylester (SE), Sulfodichlorphenyl(SDP)-Ester, Sulfotetrafluorphenyl(STP)-Ester, Tetrafluorphenyl(TFP)-Ester und Pentafluorphenyl(PFP)-Ester ausgewählt ist.

12. Verwendung eines Kits nach einem der Ansprüche 8-11, wobei das organische Lösungsmittel DMSO ist.

13. Verwendung einer Zusammensetzung zum Verfolgen der Zellproliferation in einem Verfahren nach einem der Ansprüche 1 bis 7, wobei die Zusammensetzung Folgendes umfasst:
a) eine oder mehrere der Zellverfolgungsverbindungen nach einem der Ansprüche 1 bis 3; und
b) eine Trägersubstanz oder ein Analyt,
wobei die eine oder die mehreren der Zellverfolgungsverbindungen in einer Menge vorliegen, die wirksam ist, um die Zellproliferation zu verfolgen.

14. Verwendung einer Zusammensetzung nach Anspruch 13, wobei die Zellverfolgungsverbindung an ein Trägersubstanzmolekül konjugiert ist.

15. Verwendung einer Zusammensetzung nach Anspruch 13, wobei der Analyt eine Zelle ist und sich die Zellverfolgungsverbindung innerhalb der Zelle befindet.

## Revendications

1. Procédé de suivi de la prolifération cellulaire, le procédé comprenant les étapes consistant à :
a) faire incuber un mélange de cellules avec un composé de formule développée (I) : 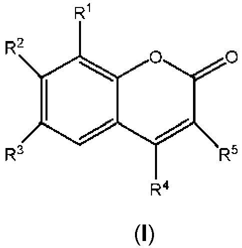 ou un sel pharmaceutiquement acceptable de celui-ci,
R¹ et R³ représentant chacun indépendamment hydrogène, halogène, alkyle ou alkyle substitué ;
R² représentant acyle, alcoxy, alcoxy substitué, amino, amino substitué, acylamino ou hydroxyle ;
R⁴ représentant hydrogène, alkyle ou alkyle substitué ;
R⁵ représentant alkyle, alkyle substitué, acyloxy, aminocarbonyle, aminocarbonylamino, aminocarbonyl-oxy, carbonyle, carboxyle, carboxylalkyle, carboxylester, (carboxylester) amino, -L-Rₓ ou - L-S_{c},
L représentant un coupleur ;
Rₓ représentant un groupe réactif ; et
S_{c} représentant une substance conjuguée ;
b) fournir un stimulus au mélange de manière à provoquer un signal fluorescent ; et
c) analyser le mélange stimulé à l'aide de la cytométrie de flux.

2. Procédé selon la revendication 1,
R¹ et R³ représentant chacun indépendamment hydrogène ou halogène ;
R² représentant acyle, alcoxy, amino, amino substitué ou hydroxyle ;
R⁴ représentant H ou alkyle ; et
R⁵ représentant -L-Rₓ,
Rₓ représentant un ester succinimidylique (SE), un ester de sulfodichlorophényle (SDP), un ester de sulfotétrafluorophényle (STP), un ester de tétrafluorophényle (TFP) ou un ester de pentafluorophényle (PFP).

3. Procédé selon la revendication 1, le composé de formule développée (I) étant choisi dans le groupe constitué par : 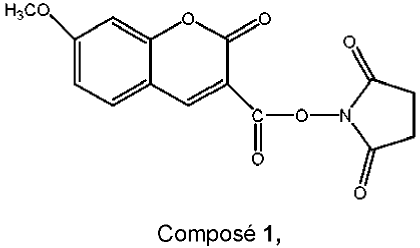 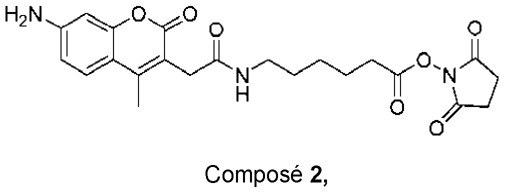 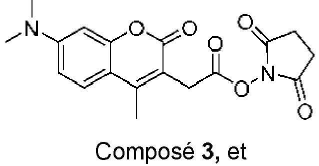 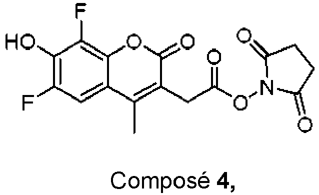 ou un sel pharmaceutiquement acceptable de celui-ci.

4. Procédé selon l'une quelconque des revendications 1 à 3, comprenant en outre un second composé pouvant être excité à une longueur d'onde différente que le composé de suivi de cellule.

5. Procédé selon la revendication 1 ou 2, Rₓ représentant un acrylamide, un acide carboxylique, un ester activé d'un acide carboxylique, un azoture d'acyle, un halogénure d'acyle, un hydroxy, un aldéhyde, un halogénure d'alkyle, un sulfonate, une aminé, un anhydride, une aniline, un halogénure d'aryle, un azide, une aziridine, un boronate, un carbodiimide, un diazoalkane, un époxyde, un glycol, un haloacétamide, une halotriazine, une hydrazine, un hydroxylamine, un imidoester, un isothiocyanate, une cétone, un maléimide, un halogénure de sulfonyle ou un groupe thiol ; ou
Rₓ représentant un acide carboxylique, un ester activé d'un acide carboxylique, une amine, un maléimide, un iodoacétamide, un isothiocyanate ou un halométhyle ; ou
Rₓ étant choisi parmi un ester succinimidylique (SE), un ester de sulfodichlorophényle (SDP), un ester de sulfotétrafluorophényle (STP), un ester de tétrafluorophényle (TFP) et un ester de pentafluorophényle (PFP).

6. Procédé selon l'une quelconque des revendications précédentes, l'étape a) étant menée pendant environ 20 minutes.

7. Procédé selon l'une quelconque des revendications précédentes, l'étape b) et l'étape c) étant effectuées simultanément.

8. Utilisation d'une trousse de suivi de la prolifération cellulaire dans un procédé selon l'une quelconque des revendications 1 à 7, la trousse comprenant :
a) un composé de formule développée **(I)** : 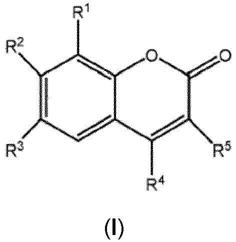 ou un sel pharmaceutiquement acceptable de celui-ci,
R¹ et R³ représentant chacun indépendamment hydrogène, halogène, alkyle ou alkyle substitué ;
R² représentant alcoxy, alcoxy substitué, amino, amino substitué, acylamino ou hydroxyle ;
R⁴ représentant hydrogène, alkyle ou alkyle substitué ;
R⁵ représentant alkyle, alkyle substitué, acyloxy, aminocarbonyle, aminocarbonylamino, aminocarbonyl-oxy, carbonyle, carboxyle, carboxylalkyle, carboxylester, (carboxylester) amino, -L-Rₓ ou -L-S_{c},
L représentant un coupleur ;
Rₓ représentant un groupe réactif ; et
S_{c} représentant une substance conjuguée ;
b) un solvant organique ; et
c) un desséchant.

9. Utilisation d'une trousse selon la revendication 8,
R¹ et R³ représentant chacun indépendamment hydrogène ou halogène ;
R² représentant acyle, alcoxy, amino, amino substitué ou hydroxyle ;
R⁴ représentant H ou alkyle ; et
R⁵ représentant -L-Rₓ,
Rₓ représentant un ester succinimidylique (SE), un ester de sulfodichlorophényle (SDP), un ester de sulfotétrafluorophényle (STP), un ester de tétrafluorophényle (TFP) ou un ester de pentafluorophényle (PFP).

10. Utilisation d'une trousse selon la revendication 8, le composé de formule développée (I) étant choisi dans le groupe constitué par : 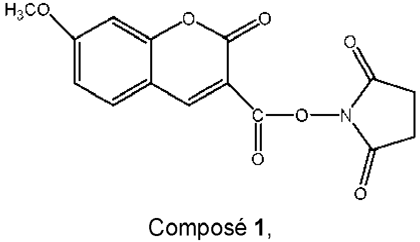 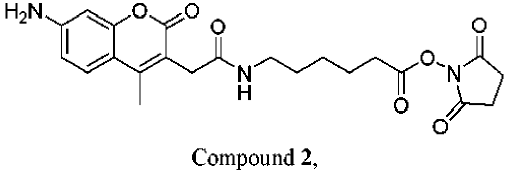 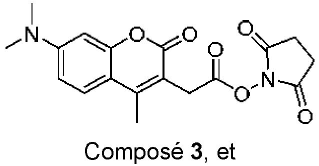 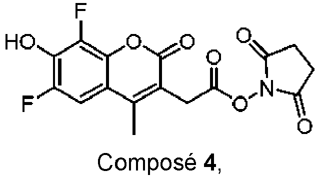 ou un sel pharmaceutiquement acceptable de celui-ci.

11. Utilisation d'une trousse selon la revendication 8 ou 9, R_{X} représentant un acrylamide, un acide carboxylique, un ester activé d'un acide carboxylique, un azoture d'acyle, un halogénure d'acyle, un hydroxy, un aldéhyde, un halogénure d'alkyle, un sulfonate, une amine, un anhydride, une aniline, un halogénure d'aryle, un azide, une aziridine, un boronate, un carbodiimide, un diazoalcane, un époxyde, un glycol, un haloacétamide, un halotriazine, une hydrazine, une hydroxylamine, un imidoester, un isothiocyanate, une cétone, un maléimide, un halogénure de sulfonyle ou un groupe thiol ; ou
R_{X} représentant un acide carboxylique, un ester activé d'un acide carboxylique, une amine, un maléimide, un iodoacétamide, un isothiocyanate ou un halométhyle ; ou
R_{X} étant choisi parmi un ester de succinimidyle (SE), un ester de sulfodichlorophényle (SDP), un ester de sulfotétrafluorophényle (STP), un ester de tétrafluorophényle (TFP) et un ester de pentafluorophényle (PFP).

12. Utilisation d'une trousse selon l'une quelconque des revendications 8 à 11, le solvant organique étant le DMSO.

13. Utilisation d'une composition pour le suivi de la prolifération cellulaire dans un procédé selon l'une quelconque des revendications 1 à 7, la composition comprenant :
a) un ou plusieurs des composés suiveurs de cellules selon l'une quelconque des revendications 1 à 3 ; et
b) un porteur ou un analyte,
le ou les composés suiveurs de cellules étant présents en une quantité efficace pour suivre la prolifération cellulaire.

14. Utilisation d'une composition selon la revendication 13, le composé suiveur de cellules étant conjugué à une molécule porteuse.

15. Utilisation d'une composition selon la revendication 13, l'analyte étant une cellule et le composé suiveur de cellules étant situé à l'intérieur de la cellule.
